# EUROPEAN PATENT APPLICATION

(11) **EP 4 779 305 A2**
(43) Date of publication of application: **22.07.2026**
(21) Application number: 26166458.5
(22) Date of filing: 12.07.2022
(51) Int. Cl.: G01N 33/48

(54) **METHODS FOR PREPARING POLYMERIZED MATRIX WITH CONTROLLABLE THICKNESS**

(30) Priority: 13.07.2021 US 202163221456 P
(62) Divisional of application: 22773568.5
(71) Applicant: 10X Genomics, Inc., Pleasanton, CA 94588-3260 (US)
(72) Inventor: CHEN, Hong, Pleasanton, CA 94588-3260 (US)
(74) Representative: J A Kemp LLP

(57) **Abstract**

The present disclosure relates in some aspects to methods for preparing a thin polymer matrix (e.g., a hydrogel matrix) having a biological sample embedded therein for *in situ* analysis of one or more analytes.

## Description

### CROSS-REFERENCE TO RELATED APPLICATION

This application claims priority to U.S. Provisional Patent Application No. 63/221,456, filed July 13, 2021, entitled "METHODS FOR PREPARING POLYMERIZED MATRIX WITH CONTROLLABLE THICKNESS," which is herein incorporated by reference in its entirety for all purposes.

### REFERENCE TO AN ELECTRONIC SEQUENCE LISTING

The contents of the electronic sequence listing (202412008940SEQLIST.xml; Size: 3,652 bytes; and Date of Creation: July 11, 2022) is herein incorporated by reference in its entirety.

### FIELD

The present disclosure relates in some aspects to methods, compositions, kits, and devices for sample processing and analysis.

### BACKGROUND

Transcription profiling of cells is essential for many purposes, such as understanding the molecular basis of cell identity and/or function and developing treatments for diseases. Microscopy imaging, which can resolve multiple mRNAs in single cells, can provide valuable information such as transcript abundance and spatial information *in situ.* Methods are available for analyzing nucleic acids present in a biological sample, such as a cell or a tissue. For instance, advances in single molecule fluorescence *in situ* hybridization (smFISH) have enabled nanoscale-resolution imaging of RNA in cells and tissues. However, existing methods of *in situ* analysis of biological samples may suffer from low signal-to-noise ratios, e.g., due to background signals such as autofluorescence in a sample. Improved methods for sample processing and *in situ* analysis are needed. The present disclosure addresses these and other needs.

### SUMMARY

In some aspects, provided herein are methods for preparing a polymer matrix (e.g., a hydrogel) with a desired thickness for sample processing and/or *in situ* analysis of one or more analytes (e.g., nucleic acid and/or protein molecules) in a biological sample embedded in the polymer matrix. In some embodiments, disclosed herein are methods of preparing three-dimensional polymerized matrices and methods of embedding biological samples in three-dimensional polymerized matrices. In some embodiments, a matrix-forming material is contacted with a biological sample, and as a three-dimensional polymerized matrix is formed from the matrix-forming material, the biological sample is embedded in the matrix. In some embodiments, the thickness of the three-dimensional polymerized matrix can be precisely controlled, e.g., to provide a hydrogel layer having a uniform thickness that is thinner than hydrogel layers generated using certain existing methods.

In some aspects, the polymerized matrix not only immobilizes the biological sample but also facilitate processing (e.g., clearing) of the embedded sample and/or detection of analytes (e.g., nucleic acid molecules) in sequential probe hybridization cycles. In some aspects, the methods provided herein employ a spacer, such as adhesive tape and/or microparticles, to controllably achieve a thin layer of matrix that in some embodiments has a thickness comparable to that of the biological sample embedded therein.

In one aspect, provided herein is a method for preparing a three-dimensional polymerized matrix, comprising: a) delivering a matrix-forming material in a space between a surface of a first planar substrate and a surface of a second planar substrate, wherein the space is at least partially enclosed by a spacer between the first and second planar substrates, and the spacer is non-integral to the first or second planar substrate; and b) forming a three-dimensional polymerized matrix from the matrix-forming material in the space, wherein the three-dimensional polymerized matrix has an average thickness between about 5 µm and about 200 µm. In some embodiments, the method further comprises c) removing the spacer from the first and/or the second planar substrates.

In any of the preceding embodiments, the spacer can comprise an adhesive tape, optionally wherein the adhesive tape has a thickness of about 100 µm or between about 10 µm and 20 µm. In any of the preceding embodiments, the distance between the surfaces of the first and second planar substrates can be substantially equal to the thickness of the adhesive tape.

In any of the preceding embodiments, the spacer can comprise a plurality of microparticles such as rigid microparticles, optionally wherein the average diameter of the microparticles is between about 10 µm and 50 µm. In any of the preceding embodiments, the distance between the surfaces of the first and second planar substrates can be substantially equal to the average diameter of the microparticles. In any of the preceding embodiments, the plurality of microparticles can be monodisperse. In any of the preceding embodiments, the plurality of microparticles can comprise a plurality of silica monospheres.

In any of the preceding embodiments, the method can further comprise applying the spacer to the surface of the first planar substrate and/or the surface of the second planar substrate, and bringing the first planar substrate and second planar substrate together such that the spacer separates the first and second planar substrates to provide the space.

In some embodiments wherein the spacer comprises a plurality of microparticles, the applying step comprises adding a suspension to the surface of either the first planar substrate or second planar substrate, wherein the suspension comprises the plurality of microparticles and a carrier. In some embodiments, the carrier comprises an oil such as a mineral oil. In some embodiments, the carrier comprises an organic solvent. In some embodiments, the carrier comprises ethanol. In some embodiments, the plurality of microparticles are not mixed with the matrix-forming material and do not contact the three-dimensional polymerized matrix.

In any of the preceding embodiments, the three-dimensional polymerized matrix can comprise a biological sample embedded therein. In any of the preceding embodiments, the biological sample can be immobilized on the surface of the first planar substrate. In any of the preceding embodiments, the method can further comprise fixing the biological sample prior to delivering the matrix-forming material. In any of the preceding embodiments, the first planar substrate can comprise a coating with one or more substances to facilitate attachment of the biological sample. In any of the preceding embodiments, the first planar substrate and/or the coating can be functionalized with one or more substances to facilitate attachment of the biological sample. In any of the preceding embodiments, the one or more substances can comprise lectins, poly-lysine, antibodies, polysaccharides, or covalently binding moieties, or any combination thereof. In any of the preceding embodiments, the one or more substances can comprise acryloyls.

In any of the preceding embodiments, the method can further comprise permeabilizing and/or fixing the biological sample prior to the delivering step. In any of the preceding embodiments, the method can further comprise cross-linking the biological sample embedded within the three-dimensional polymerized matrix. In any of the preceding embodiments, the method can further comprise clearing the biological sample embedded within the three-dimensional polymerized matrix.

In any of the preceding embodiments, the biological sample can be a tissue slice between about 1 µm and about 50 µm in thickness, optionally wherein the tissue slice is between about 5 µm and about 35 µm in thickness.

In any of the preceding embodiments, the matrix-forming material can comprise a plurality of fluorescent beads. In some embodiments, the thickness of the three-dimensional polymerized matrix is measured by imaging the fluorescent beads. In any of the preceding embodiments, the fluorescent beads can have an average diameter of about 0.2 µm.

In any of the preceding embodiments, the method can further comprise applying a force to the first and/or second planar substrates prior to forming the three-dimensional polymerized matrix. In any of the preceding embodiments, the three-dimensional polymerized matrix can be formed by subjecting the matrix-forming material to polymerization. In any of the preceding embodiments, the polymerization can be initiated by adding a polymerization-inducing catalyst, UV light or functional cross-linkers, or any combination thereof. In any of the preceding embodiments, the matrix-forming material can comprise polyacrylamide, cellulose, alginate, polyamide, cross-linked agarose, cross-linked dextran or cross-linked polyethylene glycol.

In any of the preceding embodiments, the surface of the first or second planar substrate can have a recessed cavity. In any of the preceding embodiments, the surface of the first or second planar substrate can comprise one or more positional markers and/or fiducial markers.

In another aspect, provided herein is a method for embedding a biological sample in a three-dimensional polymerized matrix, comprising: a) applying a spacer to a surface of a first planar substrate or a surface of a second planar substrate, wherein the spacer is non-integral to the first or second planar substrate, and wherein a biological sample is immobilized on the surface of a first planar substrate ; b) bringing the first and second planar substrates together to form a space between the first and second substrates such that the biological sample is in the space at least partially enclosed by the spacer; c) delivering a matrix-forming material in the space; and d) forming a three-dimensional polymerized matrix from the matrix-forming material in the space, wherein the three-dimensional polymerized matrix has an average thickness between about 10 µm and about 100 µm, thereby embedding the biological sample in the three-dimensional polymerized matrix.

In any of the preceding embodiments, the spacer can be applied to the second planar substrate which is downward facing, and the method can further comprise removing the second planar substrate from the first planar substrate, thereby removing the spacer at least partially enclosing the biological sample. In any of the preceding embodiments, the spacer can be applied to the first planar substrate which is upward facing, and the method can further comprise removing the second planar substrate from the first planar substrate, and optionally further removing the spacer from the first planar substrate.

In any of the preceding embodiments, the method can further comprise crosslinking the biological sample embedded in the three-dimensional polymerized matrix. In any of the preceding embodiments, the method can further comprise clearing the biological sample embedded within the three-dimensional polymerized matrix. In any of the preceding embodiments, the clearing can be performed for less than about 60 minutes. In any of the preceding embodiments, the clearing can be performed for less than about 30 minutes.

In yet another aspect, provided herein is a three-dimensional polymerized matrix generated according to a method disclosed herein, wherein the three-dimensional polymerized matrix has an average thickness between about 5 µm and about 200 µm.

In still yet another aspect, provided herein is a biological sample embedded in a three-dimensional polymerized matrix generated according to a method disclosed herein, wherein the three-dimensional polymerized matrix has an average thickness between about 5 µm and about 200 µm.

Also provided herein are kits for performing the methods of preparing three-dimensional polymerized matrices and/or for embedding a biological sample in a three-dimensional polymerized matrix. In one aspect, provided herein is a kit for preparing a three-dimensional polymerized matrix, comprising a first planar substrate, a second planar substrate, one or more spacers (e.g., adhesive tape or rigid microparticles), one or more matrix-forming materials (e.g., hydrogel monomers), and instructions for performing the methods provided herein.

In still other embodiments, the kits further comprise one or more reagents for performing the analytical methods provided herein. In some embodiments, the kits further comprise one or more reagents required for one or more steps comprising hybridization, ligation, extension, detection, sequencing, and/or sample preparation as described herein. In some embodiments, the kit further comprises a target nucleic acid. In some embodiments, any or all of the oligonucleotides are DNA molecules. In some embodiments, the target nucleic acid is a messenger RNA molecule. In some embodiments, the target nucleic acid is a probe (e.g., a padlock probe) or an amplification product thereof (e.g., a rolling circle amplification product, such as a nucleic acid concatemer). The various components of the kit may be present in separate containers or certain compatible components may be precombined into a single container. In some embodiments, the kits further contain instructions for using the components of the kit to practice the provided methods.

In some embodiments, the kits can contain reagents and/or consumables required for performing one or more steps of the provided methods. In some embodiments, the kits contain reagents for fixing, embedding, and/or permeabilizing the biological sample. In some embodiments, the kits contain reagents, such as enzymes and buffers for ligation and/or amplification, such as ligases and/or polymerases. In some aspects, the kit can also comprise any of the reagents described herein, e.g., wash buffer and ligation buffer. In some embodiments, the kits contain reagents for detection and/or sequencing, such as detectable probes or detectable labels. In some embodiments, the kits optionally contain other components, for example nucleic acid primers, enzymes and reagents, buffers, nucleotides, photoreactive nucleotides, and reagents for additional assays.

### BRIEF DESCRIPTION OF THE DRAWINGS

The present application can be understood by reference to the following description taken in conjunction with the accompanying figures that illustrate certain embodiments of the features and advantages of this disclosure. These embodiments are not intended to limit the scope of the appended claims in any manner.

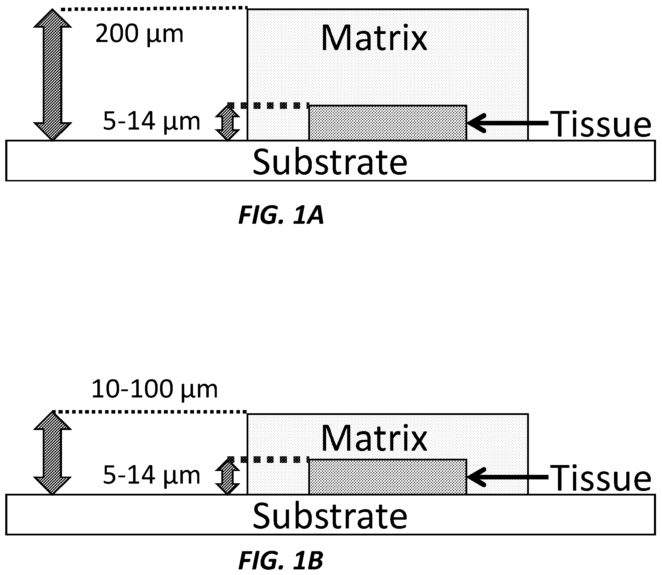

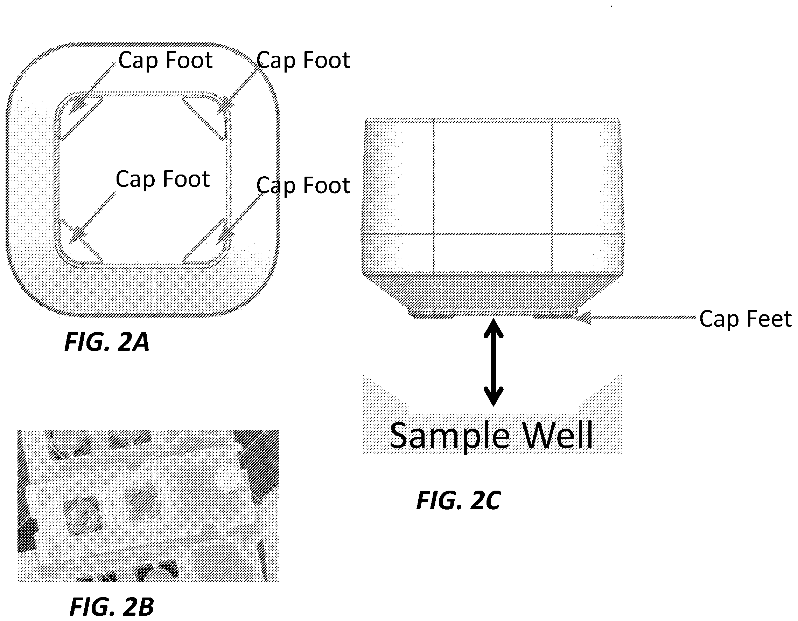

**FIGS. 1A** and **1B** depict exemplary biological samples (*e.g*., tissue samples) embedded in a three-dimensional polymerized matrix (*e.g.,* a hydrogel matrix). **FIG. 1A** shows a tissue sample embedded in a three-dimensional polymerized matrix prepared using a machined sample cap having integral cap feet (e.g., as shown in **FIG. 2A**). **FIG. 1B** shows a tissue sample embedded in a three-dimensional polymerized matrix prepared according to a method of the present disclosure. The hydrogel matrix in **FIG. 1B** has a reduced thickness (e.g., 10-100 µm) in contrast to the hydrogel matrix in **FIG. 1A** with a thickness (~200 µm) limited by engineering of the machined sample caps.
**FIGS. 2A-2C** depict different views of a polymer casting mold comprising a pedestaled casting cap and a base (e.g., sample well) for the cap. **FIG. 2A** shows a bottom view of a pedestaled casting cap, in which the arrows indicate four pedestals, or feet at the bottom of the cap. **FIG. 2B** shows an image of the base (e.g., sample well), which has a recessed area for receiving the bottom of the casting cap and for holding any tissue samples and matrix-forming materials. **FIG. 2C** shows a side view of the pedestaled casting cap and the sample well. The pedestaled casting cap is placed on top of the sample well, and matrix-forming material (*e.g*., hydrogel mixture or "gel mix") is delivered to the empty space between the sample well and the bottom of the casting cap, for example, via an injection port in the top of the casting cap. The height of the four pedestals sets the maximum thickness (~200 µm) of a three-dimensional polymerized matrix (*e.g.,* a hydrogel matrix) formed in the space between the base of the mold and the bottom of the casting cap.

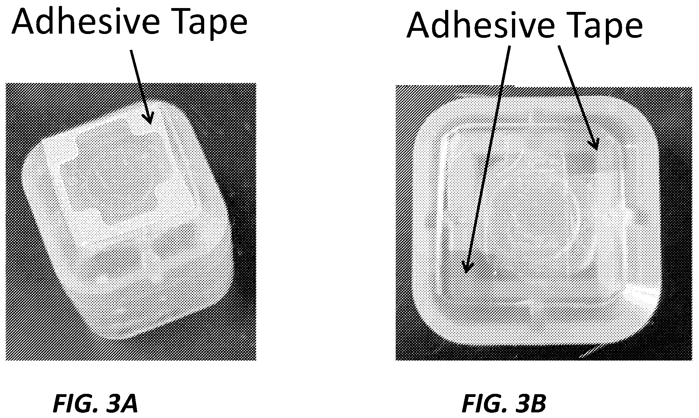

**FIGS. 3A** and **3B** depict the application of an adhesive tape in the preparation of a thin hydrogel matrix according to the present disclosure. **FIG. 3A** and **FIG. 3B** show two types of adhesive tape applied to the bottom of a flat casting cap without a pedestal. The adhesive tape replaces the pedestals, or feet, of a machined sample cap, as described for **FIGS. 2A** and **2B** above. The thickness of the adhesive tape, which sets the distance between the bottom of the casting cap (or other substrate) and a glass slide or sample well, sets the maximum thickness (on the order of 10-100 µm) of a three-dimensional polymerized matrix (*e.g.,* a hydrogel matrix).

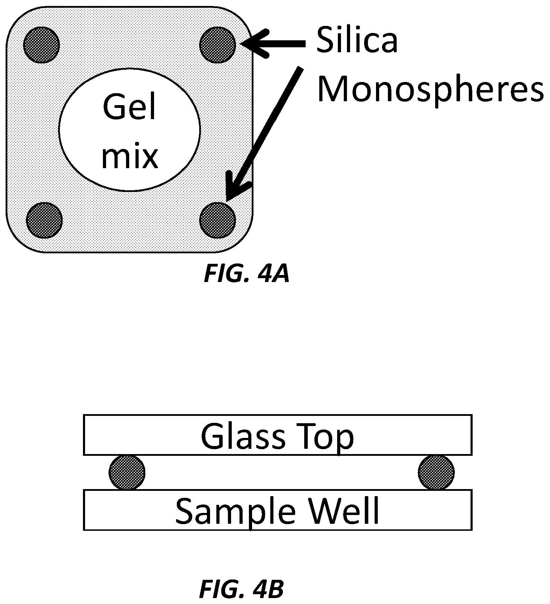

**FIGS. 4A** and **4B** depict the application of silica microparticles (*e.g*., monospheres) in the preparation of a thin hydrogel matrix according to the present disclosure.
**FIG. 4A** illustrates a top view of a sample well, which is occupied by a matrix-forming material (*e.g.,* hydrogel mixture or "gel mix") and optionally also a biological sample (*e.g*., tissue sample). In **FIG. 4A****,** the arrows indicate silica monospheres applied around the hydrogel mixture. **FIG. 2B** depicts a side view of a sample well to which silica monospheres have been applied and onto which a glass top has been placed. The average diameter of the silica monospheres sets the distance between the glass top and a glass slide or sample well, which further sets the maximum thickness (on the order of 10-50 µm) of a three-dimensional polymerized matrix (*e.g*., a hydrogel matrix).

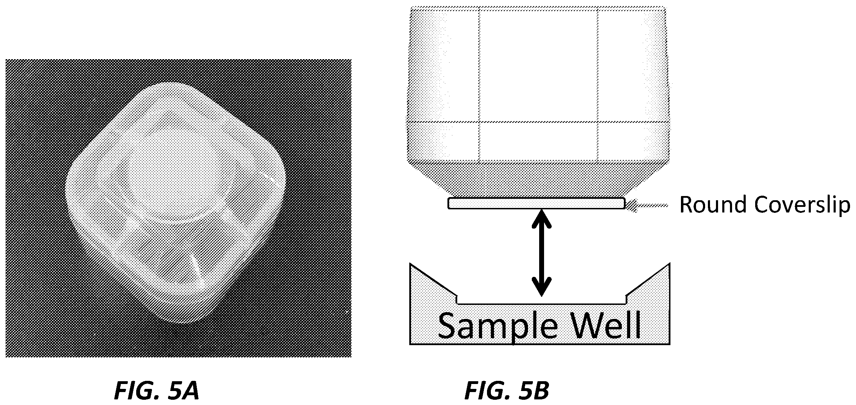

**FIGS. 5A** and **5B** depict two views of a comparative casting cap assembly, in which a flat plastic casting cap is mounted with a coverslip (*e.g.,* a #2 round coverslip with 0.0.17-0.25 mm thickness, 12 mm diameter). The coverslip is placed on the center of the bottom of the casting cap; the casting cap is placed, with the coverslip side down, atop a sample well into which the matrix-forming material is delivered. The surface tension of the gel mixture and the weight of the coverslip may be used to control the thickness of the resulting hydrogel matrix.

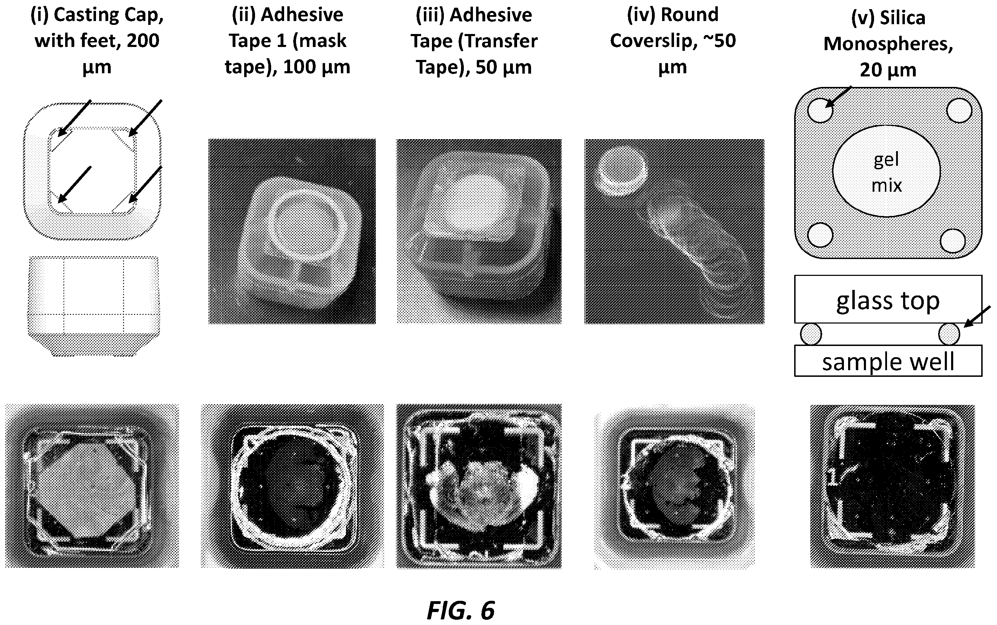

**FIG. 6** depicts a comparison of hydrogel matrices prepared by various methods as described herein, including, from left-to-right: (i) a machined casting cap with feet, 200 µm; (ii) a flat casting cap with a round coverslip and adhesive tape (laser cut), 100 µm; (iii) a flat casting cap with a round coverslip and adhesive tape (scissor cut), 50 µm; (iv) a flat casting cap with round coverslip only, 50 µm; and (v) silica monospheres, 20 µm. The middle and bottom panels depict the casting materials used (middle) and the hydrogels formed (bottom) for each of the methods indicated in above.

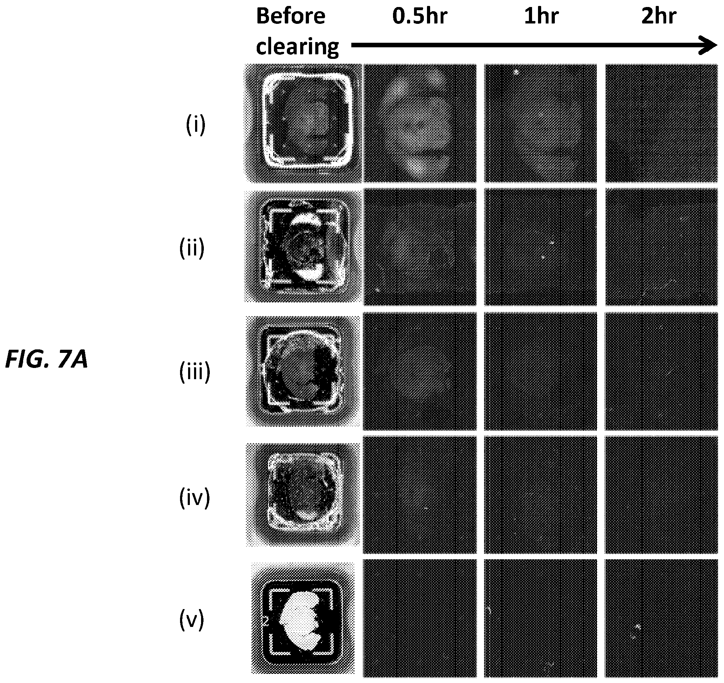

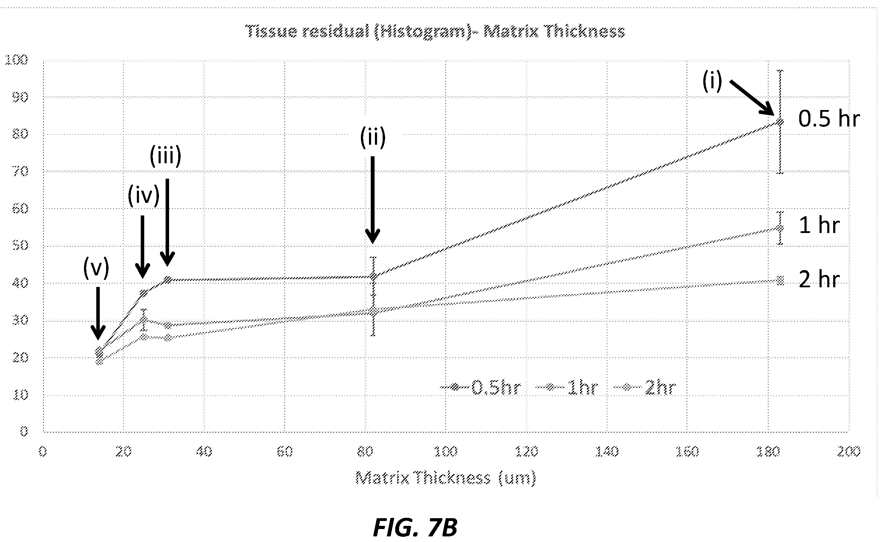

**FIGS. 7A** and **7B** depict a comparison of tissue clearing for tissue samples embedded in a hydrogel matrix, prepared with (i) a machined casting cap with pedestals, 200 µm; (ii) adhesive tape, 70-100 µm; (iii) 14-50 µm, round coverslip; (iv) silica monospheres, 20 µm; and (v) tissue sample only (no hydrogel; control). **FIG. 7A** shows fluorescent images of the tissue at various time points (before clearing, at 0.5 h, at 1 h, at 2h); **FIG. 7B** shows a plot of residual tissue (%) against initial hydrogel matrix thickness (µm); the residual tissue before clearing was 100%.

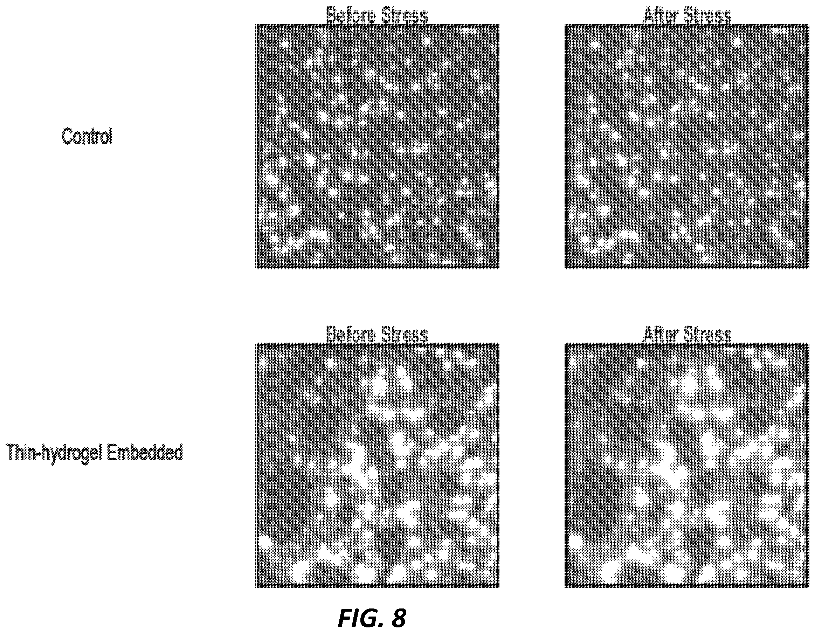

**FIG. 8** depicts exemplary images of rolling circle amplification products (RCPs) generated and detected *in situ* in a mouse brain tissue sample embedded in a thin hydrogel and in a control sample that was not hydrogel embedded. The RCPs in the embedded sample were crosslinked to the hydrogel gel matrix. Samples are depicted prior to stress (15 fluidic cycles) and after stress.

### DETAILED DESCRIPTION

All publications, comprising patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

Provided herein, in some aspects, are methods for preparing thin three-dimensional polymerized matrices (*e.g*., hydrogel matrices). In some embodiments, the methods for preparing the thin three-dimensional polymerized matrices are methods for preparing hydrogel-embedded biological samples. In certain embodiments, the hydrogel-embedded biological samples obtained according to the methods provided herein may further be used in or for methods for detecting the presence of and/or quantifying analytes, such as nucleic acids, in cells, tissues, organs or organisms. In some embodiments, the present disclosure provides methods for high-throughput profiling of a large number of targets *in situ,* including spatial information of such targets, such as RNA transcripts and/or DNA loci in a tissue sample.

### I. OVERVIEW

Prior to the imaging of such biological samples, the cells or tissues are typically embedded in three-dimensional polymerized matrices, such as hydrogel matrices, to prevent detachment from microscopy slides, to mitigate sample degradation, and to preserve relative spatial information of the tissue (*e.g.,* link any molecule of interest to its original location for colocalization with other molecules of interest). The embedding of biological samples in hydrogel matrices also allows for improvement in signal-to-noise ratios, by enabling tissue clearing to reduce autofluorescence background. However, existing methods for preparing biological samples in hydrogel matrices lead to a number of process inefficiencies, such as longer diffusion times for tissue preparation and use of excessive materials, such as hydrogel materials or reagents, to prepare and penetrate into hydrogel-embedded tissues. Furthermore, the discrepancy in thickness between the average, comparatively thin tissue sample (~5-20 µm) and the relatively large thickness of the hydrogels in which the tissue samples are embedded (~200 µm or greater) can introduce excessive background noise into imaging signals. Thus, improved methods for preparing and embedding biological samples in hydrogel matrices for *in situ* analysis are needed.

The embedding of biological samples in three-dimensional matrices, such as hydrogels, is a key step in sample preparation for stabilizing the integrity of biological samples for handling and allowing for further downstream treatment of the biological sample to facilitate analysis, such as by microscopy imaging. For example, after being embedding into a hydrogel matrix, a biological sample may be subjected to any number of post-processing steps including but not limited to clearing, cross-linking, expansion, *etc.*

More recent analytic techniques for evaluating biological samples, such as transcriptional profiling, still employ well-established histological processing steps as part of their sample preparation. Even with newer analytic techniques, standard preparation techniques for embedding such biological samples remain the norm. However, for transcriptional profiling, which involves analytes present at low concentrations, such as nucleic acids, and relies upon highly sensitive measurements, for example, single molecule fluorescence signals, standard embedding protocols and methods may be less favorable for evaluating biological samples.

For example, with reference to **FIG. 1A****,** existing methods for embedding tissue samples in hydrogel matrices or other three-dimensional polymerized matrices produce hydrogel matrices having thicknesses on the order of 200 µm. In contrast, the biological samples, which themselves are embedded in these matrices, only range in thickness from 5 µm to 20 µm. The incongruity between the thickness of the biological sample and the thickness of the hydrogel matrix encapsulating the biological sample can result in excess background noise for the measurement and analysis of the biological sample. The excess thickness may lead to additional overall inefficiencies in terms of requiring more materials for sample preparation, and increasing the time for various steps, such as diffusion time for clearing tissue.

Existing methods for preparing tissue samples rely upon the use of casting molds having engineered dimensions to control the thickness of hydrogel matrices. For example, a common casting mold may include a glass slide with an affixed biological sample or a prefabricated sample well containing a biological sample, along with a complementary casting cap having a fixed height, as shown in **FIGS. 2A-2C****.** In **FIG. 2C****,** the pedestals (or feet) present on the bottom of the casting cap control distance between the bottom of the casting cap and the surface of sample well and, thus, control the height of any hydrogel matrix formed in the space between. Hydrogel monomer mixture is injected into the void space through an injection port in the casting cap, and the mixture polymerized to form the hydrogel matrix.

While the manufacture of casting molds to having more closely matched thicknesses to those of tissue samples is feasible, such modifications would require highly expensive, precision engineering of these casting molds to achieve the micrometer or sub-micrometer heights desired while maintaining a precise surface flatness (*e.g*., ±1-2 µm) for a uniformly thick hydrogel. Furthermore, as hydrogel matrices become thinner, the tolerance for the thickness of the hydrogel becomes increasingly stringent. For example, an absolute variation of 5 µm for a 100 µm-thick hydrogel constitutes 5% variation, whereas the same absolute variation in a 15 µm-thick hydrogel represents 33% variation. Thus, there is a need for alternative methods of preparing biological samples embedded in thin, uniform hydrogel matrices for *in situ* analysis in a cost-effective manner that also gives reproducibly, precisely thin matrices on the order of single micrometers.

The present disclosure addresses this need by providing methods for preparing thin polymer matrices (e.g., hydrogels) more similar to, or even comparable to, the thickness of a typical biological sample, such as shown in **FIG. 1B****,** and, more specifically, hydrogel matrices having thicknesses in the range of tens of micrometers to hundreds of nanometers.

In some aspects, the methods of the present disclosure achieve these thicknesses of hydrogels by employing spacer materials, which may be applied to existing casting mold and/or casting caps, or any other desired planar surface or substrate, thus setting the maximum thickness of any hydrogel matrices subsequently formed.

The spacers, as provided in the present disclosure, include materials such as pre-fabricated adhesive tapes or pre-formed microparticles, such as silica monospheres. The adhesive tapes have thicknesses on the order of tens of micrometers to a few hundred micrometers, and the microparticles have controlled diameters on the order of single micrometers up to several hundreds of micrometers. The spacer materials are non-integral to any pre-machined substrate or platform, and may be removably affixed to existing molding caps, glass slides, and/or other planar substrates to be used in the sample preparation of biological samples. The spacer materials are readily available and non-reactive, thus obviating the need for costly engineering to produce casting molds having pedestals with micrometer tolerance or compatibility with existing protocols for preparation of biological samples or hydrogel matrices.

In one aspect, provided herein is a method for preparing a three-dimensional polymerized matrix, wherein the three-dimensional polymerized matrix has an average thickness between about 5 µm and about 200 µm.

In another aspect, provided herein is a method for preparing a three-dimensional polymerized matrix, comprising: a) delivering a matrix-forming material in a space between a surface of a first planar substrate and a surface of a second planar substrate, wherein the space is at least partially enclosed by a spacer between the first and second planar substrates, and the spacer is non-integral to the first or second planar substrate; and b) forming a three-dimensional polymerized matrix from the matrix-forming material in the space, wherein the three-dimensional polymerized matrix has an average thickness between about 5 µm and about 200 µm.

In still yet another aspect, provided herein is a method for embedding a biological sample in a three-dimensional polymerized matrix, comprising: a) applying a spacer to a surface of a first planar substrate or a surface of a second planar substrate, wherein the spacer is non-integral to the first or second planar substrate, and wherein a biological sample is immobilized on the surface of a first planar substrate; b) bringing the first and second planar substrates together to form a space between the first and second substrates such that the biological sample is in the space at least partially enclosed by the spacer; c) delivering a matrix-forming material in the space; and d) forming a three-dimensional polymerized matrix from the matrix-forming material in the space, wherein the three-dimensional polymerized matrix has an average thickness between about10 µm and about 100 µm, thereby embedding the biological sample in the three-dimensional polymerized matrix.

In another aspect, provided herein is a three-dimensional polymerized matrix, wherein the three-dimensional polymerized matrix has an average thickness between about 5 µm and about 200 µm. In yet another aspect, provided herein is a biological sample embedded in a three-dimensional polymerized matrix, obtained by the methods according to the present disclosure, wherein the three-dimensional polymerized matrix is a hydrogel matrix and the three-dimensional polymerized matrix has an average thickness between about 5 µm and about 200 µm.

### II. PLANAR SUBSTRATES

For analytical techniques, such as microscopy, thin, planar three-dimensional polymerized matrices offer a complementary structure to stabilize for biological samples, which are often provided in the form of planar tissue sections or slices. The methods provided herein employ two planar substrates for the preparation of thin hydrogel matrices and for embedding biological samples in said hydrogel matrices. While existing methods for preparing thin three-dimensional polymerized matrices employ prefabricated casting molds, particularly the casting cap with pedestals illustrated in **FIGS. 2A-2C****,** the methods of the present disclosure may employ any suitable planar substrates to which the spacer, such as adhesive tape or rigid microparticles, can be applied, thus increasing the ease of preparing thin three-dimensional polymerized matrices.

The methods of the present disclosure for the preparation of thin three-dimensional polymerized matrices and/or biological samples embedded within three-dimensional polymerized matrices comprise delivering a matrix-forming material in a space between a surface of a first planar substrate and a surface of a second planar substrate, wherein the space is at least partially enclosed by a spacer. The distance between the first and second planar substrates sets the maximum thickness possible for a matrix-forming material to form a three-dimensional polymerized matrix.

It should be recognized that the methods of the present disclosure, while providing a highly reproducible, cost-effective means of preparing thin hydrogels do so, in part, by allowing for a variety of planar substrates to be used with the spacer materials. For example, while existing methods require precisely engineered pre-fabricated casting molds, as shown in **FIGS. 2B** and **2C****,** in some embodiments of the methods provided herein, the first and/or second planar substrates are each independently a glass slide or a plastic planar substrate, such as a plastic casting cap without pedestals.

It should be recognized that use of ordinal terms "first" and "second" in the description of the planar substrates provided herein does not by itself connote any priority, precedence, or order of one planar substrate over another or the temporal order in which acts of a method are performed, but are used merely as labels to distinguish one planar substrate having a certain name from another planar substrate (but for use of the ordinal term) to distinguish the two planar substrates.

In some embodiments, the first planar substrate is a glass slide. In some embodiments, the second planar substrate is a glass slide. In certain embodiments, the first planar substrate and the second planar substrate are glass slides. In some embodiments, the first planar substrate is a plastic planar substrate. In some embodiments, the second planar substrate is a plastic planar substrate. In certain embodiments, the first planar substrate and the second planar substrate are plastic planar substrates. In some embodiments, the first planar substrate is a glass slide and the second planar substrate is a plastic planar substrate. In other embodiments, the first planar substrate is a plastic planar substrate and the second planar substrate is a glass slide.

In some embodiments wherein the three-dimensional polymerized matrix comprises a biological sample, the biological sample may be fixed or immobilized to one of the first planar substrate and the second planar substrate. In some embodiments, the biological sample may be fixed or immobilized to the first planar substrate. In other embodiments, the biological sample may be fixed or immobilized to the second planar substrate. In certain embodiments, the biological sample may be fixed or immobilized to the first planar substrate, wherein the first planar substrate is a glass slide. In certain embodiments, the biological sample may be fixed or immobilized to the second planar substrate, wherein the second planar substrate is a glass slide.

In further embodiments, the first and/or second planar substrate may comprise one or more chemical or physical modifications to facilitate the placement of any biological sample embedded on the surface of the first and/or second planar substrate and its positioning within the three-dimensional polymerized matrix, as well as chemical modifications to facilitate the adhesion of the three-dimensional polymerized matrix to either the first or second planar substrate. Suitable chemical or physical modifications may include but are not limited to coatings or functionalization with one or more substances, which may be adhesive or non-adhesive, and recessed cavities.

In yet other embodiments, the biological sample may be fixed or immobilized to one of the first planar substrate and the second planar substrates, wherein the surface of the first planar substrate or second planar substrate to which the biological sample is fixed or immobilized further comprises a coating or is functionalized with one or more substances to facilitate attachment or deter attachment of the biological sample to certain regions of the surface.

In some embodiments, the first planar substrate comprises a coating with or is functionalized with one or more substances to facilitate attachment of the biological sample. In other embodiments, the second planar substrate comprises a coating with or is functionalized with one or more substances to facilitate attachment of the biological sample. In certain embodiments, the one or more substances comprise lectins, poly-lysine, antibodies, polysaccharides, or covalently binding moieties, optionally wherein the one or more substances comprises acryloyls.

In some embodiments, the first planar substrate comprises a coating with or is functionalized with one or more substances to deter attachment of the biological sample. In other embodiments, the second planar substrate comprises a coating with or is functionalized with one or more substances to deter attachment of the biological sample.

In other embodiments, the surface of the first and/or second substrate further comprises a coating with or is functionalized with one or more substances to deter attachment, wherein the one or more substances to facilitate attachment and the one or more substances to deter attachment are patterned to provide an adhesive region and a non-adhesive region on the surface of the first and/or second substrate.

In some embodiments, the surface of the first or second planar substrate has a recessed cavity. Recessed cavities on the first or second planar substrate may be useful for positioning and/or centering any biological sample intended to be fixed or immobilized therein or to be embedded in the resulting three-dimensional polymerized matrix. In some embodiments wherein the three-dimensional polymerized matrix comprises a biological sample and the first planar substrate has a recessed cavity, the biological sample may be fixed or immobilized to the recessed cavity of the first planar substrate. In some embodiments wherein the three-dimensional polymerized matrix comprises a biological sample and the second planar substrate has a recessed cavity, the biological sample may be fixed or immobilized to the recessed cavity of the second planar substrate.

In yet further embodiments, the first and/or second planar substrate may comprise one or more markers, such as a laser fiducial marker, to facilitate positioning and location of the three-dimensional matrix and/or any biological sample embedded therein in any subsequent measurement and analysis, for example, in microscopy imaging. A suitable positioning marker or fiducial marker may indicate the relative spatial orientation of the biological sample to be evaluated. In some embodiments, the surface of the first or second planar substrate comprises one or more positional markers and/or fiducial markers. In some embodiments wherein the three-dimensional polymerized matrix comprises a biological sample and the biological sample is fixed or immobilized to the surface of the first planar substrate, the surface of the first planar substrate comprises one or more positional markers and/or fiducial markers. In some embodiments wherein the three-dimensional polymerized matrix comprises a biological sample and the biological sample is fixed or immobilized to the surface of the second planar substrate, the surface of the second planar substrate comprises one or more positional markers and/or fiducial markers.

### III. SPACERS

As provided in the present disclosure, the methods for preparing a three-dimensional polymerized matrices (such as hydrogel matrices) and for embedding a biological sample in a three-dimensional polymerized matrix, wherein the three-dimensional polymerized matrix have thicknesses less than about 200 µm, employ readily available spacer materials to control the thickness of the three-dimensional polymerized matrix as it is formed. In addition to the flexibility of the present methods in using any suitable planar substrates to which the spacers may be applied, the availability and ease of use of the spacers having a range of thicknesses and/or diameters further contribute to the flexibility of these methods. In some embodiments, the spacer is an adhesive tape or a plurality of microparticles.

### A. Adhesive Tape

In some embodiments, the spacer is an adhesive tape. The adhesive tape may be characterized by their chemical composition and dimensions, such as thickness. The adhesive tape to be used as a spacer as provided in the methods herein is preferably non-reactive and/or inert to any biological samples, matrix-forming materials or associated reagents for sample processing or polymerization.

In some embodiments, the adhesive tape has a thickness of about 200 µm, about 150 µm, about 100 µm, about 50 µm, about 20 µm, about 10 µm, or about 5 µm. In other embodiments, the adhesive tape has a thickness of less than or equal to about 200 µm, less than or equal to about 150 µm, less than or equal to about 100 µm, less than or equal to about 50 µm, less than or equal to about 20 µm, or less than or equal to about 10 µm. In yet other embodiments, the adhesive tape has a thickness of greater than or equal to about 100 µm, greater than or equal to about 50 µm, greater than or equal to about 20 µm, greater than or equal to about 10 µm, or greater than or equal to about 5 µm.

In some embodiments, the adhesive tape has a thickness between about 5 µm and about 200 µm, between about 5 µm and about 150 µm, between about 5 µm and about 100 µm, between about 5 µm and about 50 µm, between about 5 µm and about 20 µm, between about 5 µm and about 10 µm, between about 10 µm and about 200 µm, between about 10 µm and about 150 µm, between about 10 µm and about 100 µm, between about 10 µm and about 50 µm, between about 10 µm and about 20 µm, between about 20 µm and about 200 µm, between about 20 µm and about 150 µm, between about 20 µm and about 100 µm, or between about 20 µm and about 50 µm. In other embodiments, the adhesive tape has a thickness between about 5 µm and about 200 µm. In other embodiments, the adhesive tape has a thickness between about 10 µm and about 100 µm, between about 10 µm and about 20 µm or between about 5 µm and about 20 µm.

In some embodiments, the spacer is an adhesive tape, optionally wherein the adhesive tape has a thickness of about 100 µm or between about 10 µm and 20 µm.

### B. Plurality of Microparticles

In some embodiments, the spacer comprises a plurality of microparticles such as sufficiently rigid microparticles. The plurality of microparticles to be used as a spacer as provided in the methods herein are preferably non-reactive and/or inert to any biological samples, matrix-forming materials or associated reagents for sample processing or polymerization.

The plurality of microparticles may be characterized by their chemical composition, shape and/or dimensions, and other size distribution properties, such as average diameter or poly- or monodispersity. The plurality of microparticles to be used as a spacer as provided in the methods herein are preferably non-reactive and/or inert to any biological samples, matrix-forming materials or associated reagents for sample processing or polymerization.

In some embodiments, the plurality of microparticles comprises a plurality of rigid silica microparticles. In some embodiments, the plurality of microparticles is monodisperse. In other embodiments, the plurality of microparticles is polydisperse. In other embodiments, the plurality of microparticles comprises a plurality of silica monospheres.

In certain embodiments, the plurality of microparticles may be characterized by their size distribution, an average value for a given dimension, or a range of values for a given dimension. For example, in some embodiments, the plurality of microparticles may be characterized by an average diameter.

In some embodiments, the plurality of microparticles has an average diameter of about 200 µm, about 150 µm, about 100 µm, about 50 µm, about 20 µm, about 10 µm, or about 5 µm. In other embodiments, the plurality of microparticles has an average diameter of less than or equal to about 200 µm, less than or equal to about 150 µm, less than or equal to about 100 µm, less than or equal to about 50 µm, less than or equal to about 20 µm, or less than or equal to about 10 µm. In yet other embodiments, the plurality of microparticles has an average diameter of greater than or equal to about 100 µm, greater than or equal to about 50 µm, greater than or equal to about 20 µm, greater than or equal to about 10 µm, or greater than or equal to about 5 µm.

In some embodiments, the plurality of microparticles has an average diameter between about 5 µm and about 200 µm, between about 5 µm and about 150 µm, between about 5 µm and about 100 µm, between about 5 µm and about 50 µm, between about 5 µm and about 20 µm, between about 5 µm and about 10 µm, between about 10 µm and about 200 µm, between about 10 µm and about 150 µm, between about 10 µm and about 100 µm, between about 10 µm and about 50 µm, between about 10 µm and about 20 µm, between about 20 µm and about 200 µm, between about 20 µm and about 150 µm, between about 20 µm and about 100 µm, or between about 20 µm and about 50 µm. In other embodiments, the plurality of microparticles has an average diameter between about 5 µm and about 200 µm. In other embodiments, the plurality of microparticles has an average diameter between about 10 µm and about 100 µm, between about 10 µm and about 20 µm or between about 5 µm and about 20 µm.

In some embodiments, the spacer comprises a plurality of microparticles, optionally wherein the average diameter of the rigid microparticles is between about 10 µm and 50 µm.

In some embodiments, the plurality of microparticles are uniform in size. In some embodiments, the diameters of at least about 80%, at least about 85%, at least about 90%, at least about 95%, or at least about 99% of the microparticles are the average diameter of the plurality of microparticles. In some embodiments, the standard deviation of the diameters of the plurality of microparticles is no more than 5 µm, no more than 4 µm, no more than 3 µm, no more than 2 µm, no more than 1 µm, no more than 0.5 µm, or no more than 0.1 µm.

### C. Application of Spacers, Formation of Space Between Substrates, and/or Removal of Spacers

For the methods of the present disclosure, the spacers are easily applied to one of the first planar substrate and the second planar substrate, and optionally also easily removed from the same, which obviates the need for expensive engineering controls to pre-fabricate casting molds having precise dimensions and pre-formed spacers. As detailed above, the spacer sets the distance (or space) between the two planar substrates once they are sandwiched together, which in turn sets the maximum thickness of any three-dimensional polymerized matrix that can form in the space between the two planar substrates. In some embodiments, the spacer is removably affixed to one of the first planar substrate and the second planar substrate. The spacer is non-integral to both the first planar substrate and the second planar substrate.

In some embodiments, the methods provided herein further comprise steps for forming the space between the first and second planar substrates, including but not limited to applying a spacer to one of the first and second planar substrates and bringing together the first and second planar substrates with the spacer sandwiched between the surfaces of the first and second planar substrates, such that a space is formed between them.

In some embodiments, the methods provided herein comprise applying the spacer to the surface of the first planar substrate and/or the surface of the second planar substrate, and bringing the first planar substrate and second planar substrate together such that the spacer separates the first and second planar substrates to provide the space.

In some embodiments wherein the spacer is an adhesive tape, the applying step comprises affixing the adhesive tape to the surface of either the first planar substrate or second planar substrate.

In some embodiments wherein the spacer comprises a plurality of microparticles, the applying step comprises adding a suspension to the surface of either the first planar substrate or second planar substrate, wherein the suspension comprises the plurality of microparticles and a carrier. In some embodiments, the carrier comprises mineral oil. In some embodiments, the carrier comprises an organic solvent. In some embodiments, the carrier comprises ethanol, methanol, isopropanol, THF (tetrahydrofuran), NMP (N-Methylpyrrolidone), DMF (dimethylformamide), DMSO (dimethyl sulfoxide), DCM (dichloromethane), chloroform, or dioxane, or any combination thereof.

In some embodiments, the plurality of microparticles are not mixed with the matrix-forming material and do not contact the three-dimensional polymerized matrix. In some embodiments wherein the spacer comprises a plurality of microparticles, the step of delivering the matrix-forming material to the space between the surface of the first planar substrate and the surface of a second planar substrate comprises adding the matrix-forming material to the surface of the first planar substrate or the surface of a second planar substrate, adding a suspension to the surface of the first planar substrate or the surface of the second planar substrate to which the matrix-forming material was added, and bringing the first planar substrate and second planar substrate together such that the spacer separates the first and second planar substrates to provide the space.

In some embodiments wherein the applying step comprises adding a suspension to the surface of either the first planar substrate or second planar substrate, wherein the suspension comprises the plurality of microparticles and a carrier, the method further comprises evaporating the carrier to provide the plurality of microparticles deposited on the surface of the first planar substrate or second planar substrate.

The space produced by the sandwiching of the first and second planar substrates, and into which the matrix-forming material is delivered, is largely characterized by the dimension along the z-axis of the space, which is distance between the surfaces of the first and second planar substrates. In some embodiments, the space may be further characterized by its dimensions parallel to the surface of the two substrates, such as the dimensions in the x- and y-axes, and/or the total volume of space formed by the two planar substrates and the spacer, such as in cubic millimeters.

In some embodiments, the distance between the surfaces of the first and second planar substrates is substantially equal to the thickness of the adhesive tape or average diameter of rigid microparticles. In some embodiments, the space between the surfaces of the first and second planar substrates is substantially equal to the thickness of the adhesive tape or average diameter of rigid microparticles. In some embodiments, the distance between the surfaces of the first and second planar substrates is substantially equal to the thickness of the adhesive tape. In some embodiments, the distance between the surfaces of the first and second planar substrates is substantially equal to the average diameter of the rigid microparticles.

In some embodiments, the three-dimensional polymerized matrix comprises a biological sample or a biological sample is embedded in the three-dimensional polymerized matrix. In some embodiments wherein the three-dimensional polymerized matrix comprises a biological sample or a biological sample is embedded in the three-dimensional polymerized matrix, the space between the first planar substrate and the second planar substrate may comprise a biological sample. In some embodiments, the space between the first planar substrate and the second planar substrate comprises a biological tissue, wherein the biological sample is immobilized or affixed to the surface of one of the first and second planar substrates.

In some embodiments, the biological sample is immobilized or affixed to the surface of one of the first and second planar substrates prior to the step of delivering the matrix-forming material to the space. In other embodiments, the biological sample is immobilized or affixed to the surface of one of the first and second planar substrates prior to the step of applying the spacer material to the surface of one of the first and second planar substrates.

As described above, the spacer is non-integral to either the first or second planar substrate and is removably affixed. Following the formation of the three-dimensional polymerized matrix from the matrix-forming material, the spacer, the first planar substrate and the second planar substrate may be disassembled from their sandwich-type configuration to provide the three-dimensional polymerized matrix, or biological sample embedded in the three-dimensional polymerized matrix, for further downstream applications, such as characterization or analysis.

In some embodiments, the methods provided herein comprise removing the spacer from the first and/or the second planar substrates. In some embodiments, the spacer is applied to the second planar substrate which is downward facing, and the method further comprises removing the second planar substrate from the first planar substrate, thereby removing the spacer at least partially enclosing the biological sample. In other embodiments, the spacer is applied to the first planar substrate which is upward facing, and the method further comprises removing the second planar substrate from the first planar substrate, and optionally further removing the spacer from the first planar substrate.

### IV. THREE-DIMENSIONAL POLYMERIZED MATRICES, MATRIX-FORMING MATERIALS, AND POLYMERIZATION

As provided in the present disclosure, the methods described herein may be used to prepare thin three-dimensional polymerized matrices, particularly for the preparation of biological samples embedded within said three-dimensional polymerized matrices for subsequent *in situ* analysis.

### A. Three-Dimensional Polymerized Matrices and Matrix-Forming Materials

In some embodiments of the present disclosure, the methods provided herein are methods for preparing a three-dimensional polymerized matrix.

As described above, the three-dimensional matrices obtainable by the methods of the present disclosure can be produced with thicknesses less than those obtained by existing methods.

In some embodiments, the three-dimensional polymerized matrix has an average thickness of about 200 µm, about 150 µm, about 100 µm, about 50 µm, about 20 µm, about 10 µm, or about 5 µm. In other embodiments, the three-dimensional polymerized matrix has an average thickness of less than or equal to about 200 µm, less than or equal to about 150 µm, less than or equal to about 100 µm, less than or equal to about 50 µm, less than or equal to about 20 µm, or less than or equal to about 10 µm. In yet other embodiments, the three-dimensional polymerized matrix has an average thickness of greater than or equal to about 100 µm, greater than or equal to about 50 µm, greater than or equal to about 20 µm, greater than or equal to about 10 µm, or greater than or equal to about 5 µm.

In some embodiments, the three-dimensional polymerized matrix has an average thickness between about 5 µm and about 200 µm, between about 5 µm and about 150 µm, between about 5 µm and about 100 µm, between about 5 µm and about 50 µm, between about 5 µm and about 20 µm, between about 5 µm and about 10 µm, between about 10 µm and about 200 µm, between about 10 µm and about 150 µm, between about 10 µm and about 100 µm, between about 10 µm and about 50 µm, between about 10 µm and about 20 µm, between about 20 µm and about 200 µm, between about 20 µm and about 150 µm, between about 20 µm and about 100 µm, or between about 20 µm and about 50 µm. In other embodiments, the three-dimensional polymerized matrix has an average thickness between about 5 µm and about 200 µm. In other embodiments, the three-dimensional polymerized matrix has an average thickness between about 10 µm and about 100 µm, between about 10 µm and about 20 µm or between about 5 µm and about 20 µm.

In some aspects, the thickness of the three-dimensional polymerized matrix generated using a method disclosed herein is no more than about 200 µm, no more than about 150 µm, or no more than about 100 µm. For instance, the thickness of the polymer matrix can be between about 15 µm and about 100 µm. In some aspects, the difference between the thickness of the polymer matrix and the thickness of a tissue sample embedded in the polymer matrix is no more than about 150 µm, no more than about 125 µm, no more than about 100 µm, no more than about 90 µm, no more than about 80 µm, no more than about 70 µm, no more than about 60 µm, no more than about 50 µm, no more than about 40 µm, no more than about 30 µm, no more than about 25 µm, no more than about 20 µm, no more than about 15 µm, no more than about 10 µm, no more than about 5 µm, or no more than about 2 µm. In some aspects, the thickness of the polymer matrix is no more than 15 times, no more than 14 times, no more than 13 times, no more than 12 times, no more than 11 times, no more than 10 times, no more than 9 times, no more than 8 times, no more than 7 times, no more than 6 times, no more than 5 times, no more than 4 times, no more than 3 times, no more than 2 times, no more than 1.5 times, no more than 1.2 times, or no more than 1.1 times of the thickness of a tissue sample embedded in the polymer matrix.

As described above, the spacer sets the distance (or space) between the two planar substrates once they are sandwiched together, which in turn sets the maximum thickness of any three-dimensional polymerized matrix that can form in the space between the two planar substrates. By virtue of the spacer material, the average thickness of the three-dimensional polymerized matrix can be controlled such that the average thickness of the three-dimensional polymerized matrix is less than or equal to the distance between the two planar substrates, and by proxy, when adhesive tape is the spacer, less than or equal to the average thickness of adhesive tape, or, when rigid microparticles are the spacer, less than or equal to the average diameter of the rigid microparticles.

The three-dimensional polymerized matrix is formed from a matrix-forming material, under suitable conditions, such as polymerization conditions. Depending upon the nature and physical and chemical properties of the three-dimensional polymerized matrix desired, the matrix-forming materials may be selected accordingly.

In yet another aspect, provided herein is a three-dimensional polymerized matrix obtained according to the methods described herein. In still yet another aspect, provided herein is a biological sample embedded within a three-dimensional polymerized matrix obtained according to the methods described herein.

In some embodiments, the three-dimensional polymerized matrix is a hydrogel matrix. In some embodiments, the three-dimensional polymerized matrix comprises a biological sample embedded therein. In certain embodiments, the three-dimensional polymerized matrix is a hydrogel matrix comprising a biological sample embedded therein.

In some embodiments, the matrix-forming material comprises monomer units which are capable polymerizing to form a three-dimensional polymerized matrix. In certain embodiments, the matrix-forming material comprises hydrogel monomers, or subunits. In some embodiments, a hydrogel can include hydrogel subunits, such as, but not limited to, acrylamide, bis-acrylamide, polyacrylamide and derivatives thereof, poly(ethylene glycol) and derivatives thereof (e.g. PEG-acrylate (PEG-DA), PEG-RGD), gelatin-methacryloyl (GelMA), methacrylated hyaluronic acid (MeHA), polyaliphatic polyurethanes, polyether polyurethanes, polyester polyurethanes, polyethylene copolymers, polyamides, polyvinyl alcohols, polypropylene glycol, polytetramethylene oxide, polyvinyl pyrrolidone, polyacrylamide, poly(hydroxyethyl acrylate), and poly(hydroxyethyl methacrylate), collagen, hyaluronic acid, chitosan, dextran, agarose, gelatin, alginate, protein polymers, methylcellulose, and the like, and combinations thereof. In some embodiments, the matrix-forming material comprises polyacrylamide, cellulose, alginate, polyamide, cross-linked agarose, cross-linked dextran or cross-linked polyethylene glycol.

In some embodiments, a hydrogel includes a hybrid material, e.g., the hydrogel material includes elements of both synthetic and natural polymers. Examples of suitable hydrogels are described, for example, in U.S. Patent Nos. 6,391,937, 9,512,422, and 9,889,422, and in U.S. Patent Application Publication Nos. 2017/0253918, 2018/0052081 and 2010/0055733, the entire contents of each of which are incorporated herein by reference.

In some embodiments, the hydrogel can form the substrate. In some embodiments, the substrate includes a hydrogel and one or more second materials. In some embodiments, the hydrogel is placed on top of one or more second materials. For example, the hydrogel can be pre-formed and then placed on top of, underneath, or in any other configuration with one or more second materials. In some embodiments, hydrogel formation occurs after contacting one or more second materials during formation of the substrate. Hydrogel formation can also occur within a structure (e.g., wells, ridges, projections, and/or markings) located on a substrate.

### B. Additional Components

In some embodiments, the three-dimensional polymerized matrices of the present disclosure may comprise one or more additional components suitable for downstream processing and/or characterization.

In some embodiments, the matrix-forming material comprises a plurality of fluorescent beads. In certain embodiments, the matrix-forming material comprises a plurality of fluorescent beads, optionally wherein the fluorescent beads have an average diameter of about 0.2 µm. In some embodiments, the matrix-forming material comprises a plurality of fluorescent beads, and wherein the thickness of the hydrogel matrix is measured by imaging the fluorescent beads, optionally wherein the fluorescent beads have an average diameter of about 0.2 µm.

In some embodiments, hydrogel formation on a substrate occurs before, contemporaneously with, or after probes are provided to the sample. For example, hydrogel formation can be performed on the substrate already containing the probes.

In some embodiments, hydrogel formation occurs within a biological sample. In some embodiments, a biological sample (e.g., tissue section) is embedded in a hydrogel. In some embodiments, hydrogel subunits are infused into the biological sample, and polymerization of the hydrogel is initiated by an external or internal stimulus.

In embodiments in which a hydrogel is formed within a biological sample, functionalization chemistry can be used. In some embodiments, functionalization chemistry includes hydrogel-tissue chemistry (HTC). Any hydrogel-tissue backbone (e.g., synthetic or native) suitable for HTC can be used for anchoring biological macromolecules and modulating functionalization. Non-limiting examples of methods using HTC backbone variants include CLARITY, PACT, ExM, SWITCH and ePACT. In some embodiments, hydrogel formation within a biological sample is permanent. For example, biological macromolecules can permanently adhere to the hydrogel allowing multiple rounds of interrogation. In some embodiments, hydrogel formation within a biological sample is reversible.

In some embodiments, additional reagents are added to the hydrogel subunits before, contemporaneously with, and/or after polymerization. For example, additional reagents can include but are not limited to oligonucleotides (e.g., probes), endonucleases to fragment DNA, fragmentation buffer for DNA, DNA polymerase enzymes, dNTPs used to amplify the nucleic acid and to attach the barcode to the amplified fragments. Other enzymes can be used, including without limitation, RNA polymerase, transposase, ligase, proteinase K, and DNAse. Additional reagents can also include reverse transcriptase enzymes, including enzymes with terminal transferase activity, primers, and switch oligonucleotides. In some embodiments, optical labels are added to the hydrogel subunits before, contemporaneously with, and/or after polymerization.

In some embodiments, HTC reagents are added to the hydrogel before, contemporaneously with, and/or after polymerization. In some embodiments, a cell labelling agent is added to the hydrogel before, contemporaneously with, and/or after polymerization. In some embodiments, a cell-penetrating agent is added to the hydrogel before, contemporaneously with, and/or after polymerization.

### C. Polymerization

The present disclosure provides for the preparation of a three-dimensional polymerized matrix from a matrix-forming material. In some embodiments, the three-dimensional polymerized matrix is formed by subjecting the matrix-forming material to polymerization. Depending upon the matrix-forming material employed, the conditions for polymerization may vary accordingly across a number of variables including but not limited to polymerization reagents, reaction temperature, and reaction time. Suitable polymerization conditions for any of the matrix-forming materials as provided herein may be used for the methods of the present disclosure.

In some embodiments, the polymerization is initiated by adding a polymerization-inducing catalyst, UV light or functional cross-linkers. In some embodiments, polymerization is initiated by adding a polymerization-inducing catalyst. In some embodiments, polymerization is initiated by adding UV light. In certain embodiments, the UV light has a wavelength between 200 nm and 400 nm. In some embodiments, polymerization is initiated by adding one or more functional cross-linkers.

Although the thickness of the three-dimensional matrices is largely controlled by the dimensions of the spacer materials, constant pressure or force may be applied to the sandwiched planar substrates containing the matrix-forming material before and during the formation of the matrix in order to achieve the desired thickness of the resulting matrix. For example, the application of a force to the first and/or second planar substrates may facilitate the expulsion of excess matrix-forming material from the space between the two planar substrates, prevent any volume expansion by the matrix-forming material during the matrix formation, and maintain the desired distance between the two planar substrates to control the final thickness In some embodiments, the method comprises applying a force to the first and/or second planar substrates prior to forming the three-dimensional polymerized matrix.

### V. SAMPLES AND SAMPLE PROCESSING

### A. Embedding of biological samples

In some embodiments, the three-dimensional polymerized matrix comprises a biological sample. In some embodiments, the biological sample can be embedded in a matrix (e.g., a hydrogel matrix). Embedding the sample in this manner typically involves contacting the biological sample with a hydrogel such that the biological sample becomes surrounded by the hydrogel. For example, the sample can be embedded by contacting the sample with a suitable polymer material, and activating the polymer material to form a hydrogel. In some embodiments, the hydrogel is formed such that the hydrogel is internalized within the biological sample.

In some embodiments, the biological sample is immobilized on the surface of the first planar substrate. In some embodiments, the method comprises fixing the biological sample prior to the delivering step. In other embodiments, the method comprises permeabilizing the biological sample after the fixing step and prior to the delivering step. In some embodiments, the method comprises cross-linking the biological sample embedded within the three-dimensional polymerized matrix. In some embodiments of the methods provided herein, the method comprises clearing the biological sample embedded within the three-dimensional polymerized matrix.

In yet another aspect, provided herein is a method for embedding a biological sample in a three-dimensional polymerized matrix. In some embodiments, the method comprises applying a spacer to a surface of a first planar substrate or a surface of a second planar substrate, wherein the spacer is non-integral to the first or second planar substrate, and wherein a biological sample is immobilized on the surface of a first planar substrate. In some embodiments, the method further comprises bringing the first and second planar substrates together to form a space between the first and second substrates such that the biological sample is in the space at least partially enclosed by the spacer. In some embodiments, the method comprises delivering a matrix-forming material in the space. In still further embodiments, the method comprises forming a three-dimensional polymerized matrix from the matrix-forming material in the space, thereby embedding the biological sample in the three-dimensional polymerized matrix. In some embodiments, the three-dimensional polymerized matrix has an average thickness between about 10 µm and about 100 µm.

The composition and application of the hydrogel-matrix to a biological sample typically depends on the nature and preparation of the biological sample (e.g., sectioned, non-sectioned, type of fixation). As one example, where the biological sample is a tissue section, the hydrogel-matrix can include a monomer solution and an ammonium persulfate (APS) initiator/tetramethylethylenediamine (TEMED) accelerator solution. As another example, where the biological sample consists of cells (e.g., cultured cells or cells disassociated from a tissue sample), the cells can be incubated with the monomer solution and APS/TEMED solutions. For cells, hydrogel-matrix gels are formed in compartments, including but not limited to devices used to culture, maintain, or transport the cells. For example, hydrogel-matrices can be formed with monomer solution plus APS/TEMED added to the compartment to a depth ranging from about 0.1 µm to about 2 mm.

Additional methods and aspects of hydrogel embedding of biological samples are described for example in Chen et al., Science 347(6221):543-548, 2015, the entire contents of which are incorporated herein by reference.

### B. Samples

A sample disclosed herein can be or derived from any biological sample. Methods and compositions disclosed herein may be used for analyzing a biological sample, which may be obtained from a subject using any of a variety of techniques including, but not limited to, biopsy, surgery, and laser capture microscopy (LCM), and generally includes cells and/or other biological material from the subject. In addition to the subjects described above, a biological sample can be obtained from a prokaryote such as a bacterium, an archaea, a virus, or a viroid. A biological sample can also be obtained from non-mammalian organisms (e.g., a plant, an insect, an arachnid, a nematode, a fungus, or an amphibian). A biological sample can also be obtained from a eukaryote, such as a tissue sample, a patient derived organoid (PDO) or patient derived xenograft (PDX). A biological sample from an organism may comprise one or more other organisms or components therefrom. For example, a mammalian tissue section may comprise a prion, a viroid, a virus, a bacterium, a fungus, or components from other organisms, in addition to mammalian cells and non-cellular tissue components. Subjects from which biological samples can be obtained can be healthy or asymptomatic individuals, individuals that have or are suspected of having a disease (e.g., a patient with a disease such as cancer) or a pre-disposition to a disease, and/or individuals in need of therapy or suspected of needing therapy.

The biological sample can include any number of macromolecules, for example, cellular macromolecules and organelles (e.g., mitochondria and nuclei). The biological sample can be a nucleic acid sample and/or protein sample. The biological sample can be a carbohydrate sample or a lipid sample. The biological sample can be obtained as a tissue sample, such as a tissue section, biopsy, a core biopsy, needle aspirate, or fine needle aspirate. The sample can be a fluid sample, such as a blood sample, urine sample, or saliva sample. The sample can be a skin sample, a colon sample, a cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions. In some embodiments, the biological sample may comprise cells which are deposited on a surface.

The thickness of the tissue section can be a fraction of (e.g., less than 0.9, 0.8, 0.7, 0.6, 0.5, 0.4, 0.3, 0.2, or 0.1) the maximum cross-sectional dimension of a cell. However, tissue sections having a thickness that is larger than the maximum cross-section cell dimension can also be used. For example, cryostat sections can be used, which can be, e.g., 10-20 µm thick.

More generally, the thickness of a tissue section typically depends on the method used to prepare the section and the physical characteristics of the tissue, and therefore sections having a wide variety of different thicknesses can be prepared and used. For example, the thickness of the tissue section can be at least 0.1, 0.2, 0.3, 0.4, 0.5, 0.7, 1.0, 1.5, 2, 3, 4, 5, 6, 7, 8, 9, 10, 12, 13, 14, 15, 20, 30, 40, or 50 µm. Thicker sections can also be used if desired or convenient, e.g., at least 70, 80, 90, or 100 µm or more. Typically, the thickness of a tissue section is between 1-100 µm, 1-50 µm, 1-30 µm, 1-25 µm, 1-20 µm, 1-15 µm, 1-10 µm, 2-8 µm, 3-7 µm, or 4-6 µm, but as mentioned above, sections with thicknesses larger or smaller than these ranges can also be analyzed. In some embodiments, the biological sample is a tissue slice between about 1 µm and about 50 µm in thickness, optionally wherein the tissue slice is between about 5 µm and about 35 µm in thickness.

Cell-free biological samples can include extracellular polynucleotides. Extracellular polynucleotides can be isolated from a bodily sample, e.g., blood, plasma, serum, urine, saliva, mucosal excretions, sputum, stool, and tears.

Biological samples can be derived from a homogeneous culture or population of the subjects or organisms mentioned herein or alternatively from a collection of several different organisms, for example, in a community or ecosystem.

Biological samples can include one or more diseased cells. A diseased cell can have altered metabolic properties, gene expression, protein expression, and/or morphologic features. Examples of diseases include inflammatory disorders, metabolic disorders, nervous system disorders, and cancer. Cancer cells can be derived from solid tumors, hematological malignancies, cell lines, or obtained as circulating tumor cells.

Biological samples can also include fetal cells. For example, a procedure such as amniocentesis can be performed to obtain a fetal cell sample from maternal circulation. Sequencing of fetal cells can be used to identify any of a number of genetic disorders, including, e.g., aneuploidy such as Down's syndrome, Edwards syndrome, and Patau syndrome. Further, cell surface features of fetal cells can be used to identify any of a number of disorders or diseases.

Biological samples can also include immune cells. Sequence analysis of the immune repertoire of such cells, including genomic, proteomic, and cell surface features, can provide a wealth of information to facilitate an understanding the status and function of the immune system. By way of example, determining the status (e.g., negative or positive) of minimal residue disease (MRD) in a multiple myeloma (MM) patient following autologous stem cell transplantation is considered a predictor of MRD in the MM patient (see, e.g., U.S. Patent Application Publication No. 2018/0156784, the entire contents of which are incorporated herein by reference).

Examples of immune cells in a biological sample include, but are not limited to, B cells, T cells (e.g., cytotoxic T cells, natural killer T cells, regulatory T cells, and T helper cells), natural killer cells, cytokine induced killer (CIK) cells, myeloid cells, such as granulocytes (basophil granulocytes, eosinophil granulocytes, neutrophil granulocytes/hypersegmented neutrophils), monocytes/macrophages, mast cells, thrombocytes/megakaryocytes, and dendritic cells.

Biological samples can include analytes (e.g., protein, RNA, and/or DNA) embedded in a 3D matrix. In some embodiments, amplicons (e.g., rolling circle amplification products) derived from or associated with analytes (e.g., protein, RNA, and/or DNA) can be embedded in a 3D matrix. In some embodiments, a 3D matrix may comprise a network of natural molecules and/or synthetic molecules that are chemically and/or enzymatically linked, e.g., by crosslinking. In some embodiments, a 3D matrix may comprise a synthetic polymer. In some embodiments, a 3D matrix comprises a hydrogel.

In some embodiments, a substrate herein can be any support that is insoluble in aqueous liquid and which allows for positioning of biological samples, analytes, features, and/or reagents (e.g., probes) on the support. In some embodiments, a biological sample can be attached to a substrate. Attachment of the biological sample can be irreversible or reversible, depending upon the nature of the sample and subsequent steps in the analytical method. In certain embodiments, the sample can be attached to the substrate reversibly by applying a suitable polymer coating to the substrate, and contacting the sample to the polymer coating. The sample can then be detached from the substrate, e.g., using an organic solvent that at least partially dissolves the polymer coating. Hydrogels are examples of polymers that are suitable for this purpose.

In some embodiments, the substrate can be coated or functionalized with one or more substances to facilitate attachment of the sample to the substrate. Suitable substances that can be used to coat or functionalize the substrate include, but are not limited to, lectins, poly-lysine, antibodies, and polysaccharides.

A variety of steps can be performed to prepare or process a biological sample for and/or during an assay. Except where indicated otherwise, the preparative or processing steps described below can generally be combined in any manner and in any order to appropriately prepare or process a particular sample for and/or analysis.

### C. Pre-Embedding and Post-Embedding Steps

In some embodiments, the methods provided herein for preparing a three-dimensional matrix and/or embedding a biological sample in a three-dimensional matrix may further comprise additional steps to obtain and/or prepare the biological samples prior to forming the three-dimensional matrix or embedding the biological sample in the three-dimensional matrix. It should be recognized that the biological samples as provided herein may be subjected to various processing steps, the combinations of which are used to obtain and applied to each biological sample may vary depending upon the type of biological sample, the source of the biological sample and the intended analytical method for the biological sample, among others. Relevant processing steps may include but are not limited to tissue sectioning, freezing, fixation and post-fixation, permeabilizing, staining, expansion, cross-linking, de-cross-linking, and/or clearing.

In some embodiments, the methods provided herein for preparing a three-dimensional matrix and/or embedding a biological sample in a three-dimensional matrix may further comprise additional steps to prepare the biological samples after to forming the three-dimensional matrix or embedding the biological sample in the three-dimensional matrix. As with pre-processing steps applied to the biological samples, it should be recognized that the biological samples as provided herein may be subjected to various processing steps, the combinations of which are used to applied to each biological sample embedded in the three-dimensional polymerized matrix may vary depending upon the type of biological sample, the source of the biological sample and the intended analytical method for the biological sample, among others. Relevant processing steps may include but are not limited to fixation and post-fixation, permeabilizing, staining, expansion, cross-linking, de-cross-linking, and/or clearing.

In some embodiments, one or more processing steps disclosed in this section, e.g., in sections V-C-(i) to V-C-(ix), can be performed prior to, during, and/or after the embedding step.

### (i) Tissue Sectioning

In some embodiments, the method further comprises obtaining a biological sample to be embedded in the three-dimensional polymerized matrix.

A biological sample can be harvested from a subject (e.g., via surgical biopsy, whole subject sectioning) or grown in vitro on a growth substrate or culture dish as a population of cells, and prepared for analysis as a tissue slice or tissue section. Grown samples may be sufficiently thin for analysis without further processing steps. Alternatively, grown samples, and samples obtained via biopsy or sectioning, can be prepared as thin tissue sections using a mechanical cutting apparatus such as a vibrating blade microtome. As another alternative, in some embodiments, a thin tissue section can be prepared by applying a touch imprint of a biological sample to a suitable substrate material.

The thickness of the tissue section can be a fraction of (e.g., less than 0.9, 0.8, 0.7, 0.6, 0.5, 0.4, 0.3, 0.2, or 0.1) the maximum cross-sectional dimension of a cell. However, tissue sections having a thickness that is larger than the maximum cross-section cell dimension can also be used. For example, cryostat sections can be used, which can be, e.g., 10-20 µm thick.

More generally, the thickness of a tissue section typically depends on the method used to prepare the section and the physical characteristics of the tissue, and therefore sections having a wide variety of different thicknesses can be prepared and used. For example, the thickness of the tissue section can be at least 0.1, 0.2, 0.3, 0.4, 0.5, 0.7, 1.0, 1.5, 2, 3, 4, 5, 6, 7, 8, 9, 10, 12, 13, 14, 15, 20, 30, 40, or 50 µm. Thicker sections can also be used if desired or convenient, e.g., at least 70, 80, 90, or 100 µm or more. Typically, the thickness of a tissue section is between 1-100 µm, 1-50 µm, 1-30 µm, 1-25 µm, 1-20 µm, 1-15 µm, 1-10 µm, 2-8 µm, 3-7 µm, or 4-6 µm, but as mentioned above, sections with thicknesses larger or smaller than these ranges can also be analysed.

Multiple sections can also be obtained from a single biological sample. For example, multiple tissue sections can be obtained from a surgical biopsy sample by performing serial sectioning of the biopsy sample using a sectioning blade. Spatial information among the serial sections can be preserved in this manner, and the sections can be analysed successively to obtain three-dimensional information about the biological sample.

### (ii) Freezing

In some embodiments, the biological sample (e.g., a tissue section as described above) can be prepared by deep freezing at a temperature suitable to maintain or preserve the integrity (e.g., the physical characteristics) of the tissue structure. Such a temperature can be, e.g., less than -20°C, or less than -25°C, -30°C, -40°C, -50°C, -60°C, -70°C, -80°C -90°C, - 100°C, -110°C, -120°C, -130°C, -140°C, -150°C, -160°C, -170°C, -180°C, -190°C, or - 200°C. The frozen tissue sample can be sectioned, e.g., thinly sliced, onto a substrate surface using any number of suitable methods. For example, a tissue sample can be prepared using a chilled microtome (e.g., a cryostat) set at a temperature suitable to maintain both the structural integrity of the tissue sample and the chemical properties of the nucleic acids in the sample. Such a temperature can be, e.g., less than -15°C, less than -20°C, or less than -25°C.

### (iii) Fixation and Postfixation

In some embodiments, the method comprises fixing the biological sample prior to delivering matrix-forming material in the space between the first and second planar substrates.

In some embodiments, the biological sample can be prepared using formalin-fixation and paraffin-embedding (FFPE), which are established methods. In some embodiments, cell suspensions and other non-tissue samples can be prepared using formalin-fixation and paraffin-embedding. Following fixation of the sample and embedding in a paraffin or resin block, the sample can be sectioned as described above. Prior to analysis, the paraffin-embedding material can be removed from the tissue section (e.g., deparaffinization) by incubating the tissue section in an appropriate solvent (e.g., xylene) followed by a rinse (e.g., 99.5% ethanol for 2 minutes, 96% ethanol for 2 minutes, and 70% ethanol for 2 minutes).

As an alternative to formalin fixation described above, a biological sample can be fixed in any of a variety of other fixatives to preserve the biological structure of the sample prior to analysis. For example, a sample can be fixed via immersion in ethanol, methanol, acetone, paraformaldehyde (PFA)-Triton, and combinations thereof.

In some embodiments, acetone fixation is used with fresh frozen samples, which can include, but are not limited to, cortex tissue, mouse olfactory bulb, human brain tumor, human post-mortem brain, and breast cancer samples. When acetone fixation is performed, pre- permeabilization steps (described below) may not be performed. Alternatively, acetone fixation can be performed in conjunction with permeabilization steps.

In some embodiments, the methods provided herein comprises one or more post-fixing (also referred to as postfixation) steps. In some embodiments, one or more post-fixing step is performed after contacting a sample with a polynucleotide disclosed herein, e.g., one or more probes such as a circular or padlock probe. In some embodiments, one or more post-fixing step is performed after a hybridization complex comprising a probe and a target is formed in a sample. In some embodiments, one or more post-fixing step is performed prior to a ligation reaction disclosed herein, such as the ligation to circularize a padlock probe.

In some embodiments, one or more post-fixing step is performed after contacting a sample with a binding or labelling agent (e.g., an antibody or antigen binding fragment thereof) for a non-nucleic acid analyte such as a protein analyte. The labelling agent can comprise a nucleic acid molecule (e.g., reporter oligonucleotide) comprising a sequence corresponding to the labelling agent and therefore corresponds to (e.g., uniquely identifies) the analyte. In some embodiments, the labelling agent can comprise a reporter oligonucleotide comprising one or more barcode sequences.

A post-fixing step may be performed using any suitable fixation reagent disclosed herein, for example, 3% (w/v) paraformaldehyde in DEPC-PBS.

### (iv) Disaggregation of Cells

In some embodiments, the biological sample corresponds to cells (e.g., derived from a cell culture, a tissue sample, or cells deposited on a surface). In a cell sample with a plurality of cells, individual cells can be naturally unaggregated. For example, the cells can be derived from a suspension of cells and/or disassociated or disaggregated cells from a tissue or tissue section.

Alternatively, the cells in the sample may be aggregated, and may be disaggregated into individual cells using, for example, enzymatic or mechanical techniques. Examples of enzymes used in enzymatic disaggregation include, but are not limited to, dispase, collagenase, trypsin, and combinations thereof. Mechanical disaggregation can be performed, for example, using a tissue homogenizer. The biological sample may comprise disaggregated cells (e.g., nonadherent or suspended cells) which are deposited on a surface and subjected to an *in situ* assay and a spatial assay disclosed herein.

### (v) Tissue Permeabilization and Treatment

In some embodiments, the method comprises permeabilizing the biological sample after the fixing step and prior to the delivering of the matrix-forming material to the space between the first planar substrate and the second planar substrate.

In some embodiments, a biological sample can be permeabilized to facilitate transfer of analytes out of the sample, and/or to facilitate transfer of species (such as probes) into the sample. If a sample is not permeabilized sufficiently, the amount of analyte captured from the sample may be too low to enable adequate analysis. Conversely, if the tissue sample is too permeable, the relative spatial relationship of the analytes within the tissue sample can be lost. Hence, a balance between permeabilizing the tissue sample enough to obtain good signal intensity while still maintaining the spatial resolution of the analyte distribution in the sample is desirable.

In general, a biological sample can be permeabilized by exposing the sample to one or more permeabilizing agents. Suitable agents for this purpose include, but are not limited to, organic solvents (e.g., acetone, ethanol, and methanol), cross-linking agents (e.g., paraformaldehyde), detergents (e.g., saponin, Triton X-100^{™} or Tween-20^{™}), and enzymes (e.g., trypsin, proteases). In some embodiments, the biological sample can be incubated with a cellular permeabilizing agent to facilitate permeabilization of the sample. Additional methods for sample permeabilization are described, for example, in Jamur et al., Method Mol. Biol. 588:63-66, 2010, the entire contents of which are incorporated herein by reference. Any suitable method for sample permeabilization can generally be used in connection with the samples described herein.

In some embodiments, where a diffusion-resistant medium is used to limit migration of analytes or other species during the analytical procedure, the diffusion-resistant medium can include at least one permeabilization reagent. For example, the diffusion-resistant medium can include wells (e.g., micro-, nano-, or picowells) containing a permeabilization buffer or reagents. In some embodiments, where the diffusion-resistant medium is a hydrogel, the hydrogel can include a permeabilization buffer. In some embodiments, the hydrogel is soaked in permeabilization buffer prior to contacting the hydrogel with a sample. In some embodiments, the hydrogel or other diffusion-resistant medium can contain dried reagents or monomers to deliver permeabilization reagents when the diffusion-resistant medium is applied to a biological sample. In some embodiments, the diffusion-resistant medium, (i.e. hydrogel) is covalently attached to a solid substrate (i.e. an acrylated glass slide). In some embodiments, the hydrogel can be modified to both contain capture probes and deliver permeabilization reagents. For example, a hydrogel film can be modified to include spatially-barcoded capture probes. The spatially-barcoded hydrogel film is then soaked in permeabilization buffer before contacting the spatially-barcoded hydrogel film to the sample. The spatially-barcoded hydrogel film thus delivers permeabilization reagents to a sample surface in contact with the spatially-barcoded hydrogel, enhancing analyte migration and capture. In some embodiments, the spatially-barcoded hydrogel is applied to a sample and placed in a permeabilization bulk solution. In some embodiments, the hydrogel film soaked in permeabilization reagents is sandwiched between a sample and a spatially-barcoded array. In some embodiments, target analytes are able to diffuse through the permeabilizing reagent soaked hydrogel and hybridize or bind the capture probes on the other side of the hydrogel. In some embodiments, the thickness of the hydrogel is proportional to the resolution loss. In some embodiments, wells (e.g., micro-, nano-, or picowells) can contain spatially-barcoded capture probes and permeabilization reagents and/or buffer. In some embodiments, spatially-barcoded capture probes and permeabilization reagents are held between spacers. In some embodiments, the sample is punch, cut, or transferred into the well, wherein a target analyte diffuses through the permeabilization reagent/buffer and to the spatially-barcoded capture probes. In some embodiments, resolution loss may be proportional to gap thickness (e.g. the amount of permeabilization buffer between the sample and the capture probes). In some embodiments, the diffusion-resistant medium (e.g. hydrogel) is between approximately 50-500 micrometers thick including 500, 450, 400, 350, 300, 250, 200, 150, 100, or 50 micrometers thick, or any thickness within 50 and 500 micrometers.

In some embodiments, permeabilization solution can be delivered to a sample through a porous membrane. In some embodiments, a porous membrane is used to limit diffusive analyte losses, while allowing permeabilization reagents to reach a sample. Membrane chemistry and pore size can be manipulated to minimize analyte loss. In some embodiments, the porous membrane may be made of glass, silicon, paper, hydrogel, polymer monoliths, or other material. In some embodiments, the material may be naturally porous. In some embodiments, the material may have pores or wells etched into solid material. In some embodiments, the permeabilization reagents are flowed through a microfluidic chamber or channel over the porous membrane. In some embodiments, the flow controls the sample's access to the permeabilization reagents. In some embodiments, a porous membrane is sandwiched between a spatially-barcoded array and the sample, wherein permeabilization solution is applied over the porous membrane. The permeabilization reagents diffuse through the pores of the membrane and into the tissue.

In some embodiments, the biological sample can be permeabilized by adding one or more lysis reagents to the sample. Examples of suitable lysis agents include, but are not limited to, bioactive reagents such as lysis enzymes that are used for lysis of different cell types, e.g., gram positive or negative bacteria, plants, yeast, mammalian, such as lysozymes, achromopeptidase, lysostaphin, labiase, kitalase, lyticase, and a variety of other commercially available lysis enzymes.

Other lysis agents can additionally or alternatively be added to the biological sample to facilitate permeabilization. For example, surfactant-based lysis solutions can be used to lyse sample cells. Lysis solutions can include ionic surfactants such as, for example, sarcosyl and sodium dodecyl sulfate (SDS). More generally, chemical lysis agents can include, without limitation, organic solvents, chelating agents, detergents, surfactants, and chaotropic agents.

In some embodiments, the biological sample can be permeabilized by non-chemical permeabilization methods. Non-chemical permeabilization methods that can be used include, but are not limited to, physical lysis techniques such as electroporation, mechanical permeabilization methods (e.g., bead beating using a homogenizer and grinding balls to mechanically disrupt sample tissue structures), acoustic permeabilization (e.g., sonication), and thermal lysis techniques such as heating to induce thermal permeabilization of the sample.

Additional reagents can be added to a biological sample to perform various functions prior to analysis of the sample. In some embodiments, DNase and RNase inactivating agents or inhibitors such as proteinase K, and/or chelating agents such as EDTA, can be added to the sample. For example, a method disclosed herein may comprise a step for increasing accessibility of a nucleic acid for binding, e.g., a denaturation step to opening up DNA in a cell for hybridization by a probe. For example, proteinase K treatment may be used to free up DNA with proteins bound thereto.

### (vi) Staining

To facilitate visualization, biological samples can be stained using a wide variety of stains and staining techniques. In some embodiments, for example, a sample can be stained using any number of stains, including but not limited to, acridine orange, Bismarck brown, carmine, coomassie blue, cresyl violet, DAPI, eosin, ethidium bromide, acid fuchsine, haematoxylin, Hoechst stains, iodine, methyl green, methylene blue, neutral red, Nile blue, Nile red, osmium tetroxide, propidium iodide, rhodamine, or safranine.

The sample can be stained using hematoxylin and eosin (H&E) staining techniques, using Papanicolaou staining techniques, Masson's trichrome staining techniques, silver staining techniques, Sudan staining techniques, and/or using Periodic Acid Schiff (PAS) staining techniques. PAS staining is typically performed after formalin or acetone fixation. In some embodiments, the sample can be stained using Romanowsky stain, including Wright's stain, Jenner's stain, Can-Grunwald stain, Leishman stain, and Giemsa stain.

In some embodiments, biological samples can be destained. Methods of destaining or discoloring a biological sample generally depend on the nature of the stain(s) applied to the sample. For example, in some embodiments, one or more immunofluorescent stains are applied to the sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., J. Histochem. Cytochem. 2017; 65(8): 431-444, Lin et al., Nat Commun. 2015; 6:8390, Pirici et al., J. Histochem. Cytochem. 2009; 57:567-75, and Glass et al., J. Histochem. Cytochem. 2009; 57:899-905, the entire contents of each of which are incorporated herein by reference.

### (vii) Isometric Expansion

In some embodiments, a biological sample embedded in a matrix (e.g., a hydrogel) can be isometrically expanded. Isometric expansion methods that can be used include hydration, a preparative step in expansion microscopy, as described in Chen et al., Science 347(6221):543-548, 2015.

Isometric expansion can be performed by anchoring one or more components of a biological sample to a gel, followed by gel formation, proteolysis, and swelling. In some embodiments, analytes in the sample, products of the analytes, and/or probes associated with analytes in the sample can be anchored to the matrix (e.g., hydrogel). Isometric expansion of the biological sample can occur prior to immobilization of the biological sample on a substrate, or after the biological sample is immobilized to a substrate. In some embodiments, the isometrically expanded biological sample can be removed from the substrate prior to contacting the substrate with probes disclosed herein.

In general, the steps used to perform isometric expansion of the biological sample can depend on the characteristics of the sample (e.g., thickness of tissue section, fixation, cross-linking), and/or the analyte of interest (e.g., different conditions to anchor RNA, DNA, and protein to a gel).

In some embodiments, proteins in the biological sample are anchored to a swellable gel such as a polyelectrolyte gel. An antibody can be directed to the protein before, after, or in conjunction with being anchored to the swellable gel. DNA and/or RNA in a biological sample can also be anchored to the swellable gel via a suitable linker. Examples of such linkers include, but are not limited to, 6-((Acryloyl)amino) hexanoic acid (Acryloyl-X SE) (available from ThermoFisher, Waltham, MA), Label-IT Amine (available from MirusBio, Madison, WI) and Label X (described for example in Chen et al., Nat. Methods 13:679-684, 2016, the entire contents of which are incorporated herein by reference).

Isometric expansion of the sample can increase the spatial resolution of the subsequent analysis of the sample. The increased resolution in spatial profiling can be determined by comparison of an isometrically expanded sample with a sample that has not been isometrically expanded.

In some embodiments, a biological sample is isometrically expanded to a size at least 2x, 2.1x, 2.2x, 2.3x, 2.4x, 2.5x, 2.6x, 2.7x, 2.8x, 2.9x, 3x, 3.1x, 3.2x, 3.3x, 3.4x, 3.5x, 3.6x, 3.7x, 3.8x, 3.9x, 4x, 4.1x, 4.2x, 4.3x, 4.4x, 4.5x, 4.6x, 4.7x, 4.8x, or 4.9x its non-expanded size. In some embodiments, the sample is isometrically expanded to at least 2x and less than 20x of its non-expanded size.

### (viii) Crosslinking and De-crosslinking

In some embodiments, the methods provided herein comprise crosslinking the biological sample embedded in the three-dimensional polymerized matrix.

In some embodiments, the biological sample is reversibly cross-linked prior to or during an *in situ* assay round. In some aspects, the analytes, polynucleotides and/or amplification product (e.g., amplicon) of an analyte or a probe bound thereto can be anchored to a polymer matrix. For example, the polymer matrix can be a hydrogel. In some embodiments, one or more of the polynucleotide probe(s) and/or amplification product (e.g., amplicon) thereof can be modified to contain functional groups that can be used as an anchoring site to attach the polynucleotide probes and/or amplification product to a polymer matrix. In some embodiments, a modified probe comprising oligo dT may be used to bind to mRNA molecules of interest, followed by reversible crosslinking of the mRNA molecules.

In some embodiments, the biological sample is immobilized in a hydrogel via cross-linking of the polymer material that forms the hydrogel. Cross-linking can be performed chemically and/or photochemically, or alternatively by any other hydrogel-formation method. A hydrogel may include a macromolecular polymer gel including a network. Within the network, some polymer chains can optionally be cross-linked, although cross-linking does not always occur.

Where the substrate includes a gel (e.g., a hydrogel or gel matrix), oligonucleotides within the gel can attach to the substrate. The terms "hydrogel" and "hydrogel matrix" are used interchangeably herein to refer to a macromolecular polymer gel including a network. Within the network, some polymer chains can optionally be cross-linked, although cross-linking does not always occur.

In some embodiments, the substrate has a conditionally removable coating (e.g., a coating that can be removed from the surface of a substrate upon application of a releasing agent). In some embodiments, a conditionally removable coating includes a hydrogel as described herein, e.g., a hydrogel including a polypeptide-based material. Non-limiting examples of a hydrogel featuring a polypeptide-based material include a synthetic peptide-based material featuring a combination of spider silk and a trans-membrane segment of human muscle L-type calcium channel (e.g., PEPGEL^{®}), an amphiphilic 16 residue peptide containing a repeating arginine-alanine-aspartate-alanine sequence
(RADARADARADARADA, SEQ ID NO: 1) (e.g., PURAMATRIX^{®}), EAK16 (AEAEAKAKAEAEAKAK, SEQ ID NO: 2), KLD12 (KLDLKLDLKLDL, SEQ ID NO: 3), and PGMATRIX^{™}.

In some embodiments, the hydrogel in the conditionally removable coating is a stimulus-responsive hydrogel. A stimulus-responsive hydrogel can undergo a gel-to-solution and/or gel-to-solid transition upon application of one or more external triggers (e.g., a releasing agent). See, e.g., Willner, Acc. Chem. Res. 50:657-658, 2017, which is incorporated herein by reference in its entirety. Non-limiting examples of a stimulus-responsive hydrogel include a thermoresponsive hydrogel, a pH-responsive hydrogel, a light-responsive hydrogel, a redox-responsive hydrogel, an analyte-responsive hydrogel, or a combination thereof. In some embodiments, a stimulus-responsive hydrogel can be a multi-stimuli-responsive hydrogel.

A "releasing agent" or "external trigger" is an agent that allows for the removal of a conditionally removable coating from a substrate when the releasing agent is applied to the conditionally removable coating. An external trigger or releasing agent can include physical triggers such as thermal, magnetic, ultrasonic, electrochemical, and/or light stimuli as well as chemical triggers such as pH, redox reactions, supramolecular complexes, and/or biocatalytically driven reactions. See e.g., Echeverria, et al., Gels (2018), 4, 54; doi:10.3390/gels4020054, which is incorporated herein by reference in its entirety. The type of "releasing agent" or "external trigger" can depend on the type of conditionally removable coating. For example, a conditionally removable coating featuring a redox-responsive hydrogel can be removed upon application of a releasing agent that includes a reducing agent such as dithiothreitol (DTT). As another example, a pH-responsive hydrogel can be removed upon the application of a releasing agent that changes the pH.

In some embodiments, a method disclosed herein comprises de-crosslinking the reversibly cross-linked biological sample. In some embodiments, the de-crosslinking is performed prior to the spatial assay. The de-crosslinking does not need to be complete. In some embodiments, only a portion of crosslinked molecules in the reversibly cross-linked biological sample are de-crosslinked and allowed to migrate.

### (ix) Clearing

In some embodiments of the methods provided herein, the method comprises clearing the biological sample embedded within the three-dimensional polymerized matrix.

Hydrogels embedded within biological samples can be cleared using any suitable method. For example, electrophoretic tissue clearing methods can be used to remove biological macromolecules from the hydrogel-embedded sample. In some embodiments, a hydrogel-embedded sample is stored before or after clearing of hydrogel, in a medium (e.g., a mounting medium, methylcellulose, or other semi-solid mediums).

As described herein, the methods of the present disclosure can provided three-dimensional polymerized matrices comprising a biological sample, or biological samples embedded in three-dimensional polymerized matrices, wherein the three-dimensional polymerized matrix has a thickness that is significantly reduced (*e.g.,* less than or equal to about 200 µm) relative to the thickness of similar three-dimensional polymerized matrices, such as hydrogels, prepared using existing methods and apparatus. Due to the extreme thinness of the three-dimensional matrices obtained by the present methods, the time required for clearing of biological samples embedded therein is also reduced. In some embodiments, the clearing is performed for less than about 120 minutes, less than about 60 minutes, less than about 45 minutes, or less than about 30 minutes. In other embodiments, the clearing is performed for less than about 60 minutes or less than about 30 minutes.

### VI. CHARACTERIZATION, DETECTION AND ANALYSIS

In some embodiments, a three-dimensional polymerized matrix disclosed herein and/or a biological sample embedded in the three-dimensional polymerized matrix may be subjected to further characterization, analyte detection and/or analysis.

### A. Characterization of Three-Dimensional Polymerized Matrix

As described herein, the methods of the present disclosure provide three-dimensional polymerized matrices or biological samples embedded in three-dimensional polymerized matrices, wherein the three-dimensional polymerized matrices have an average thickness of between about 5 µm and about 200 µm.

In some embodiments, the methods of the present disclosure further comprise measuring the thickness of the three-dimensional polymerized matrix. In some embodiments, the matrix-forming material comprises a plurality of fluorescent beads, and the thickness of the three-dimensional polymerized matrix is measured by imaging the fluorescent beads. In some embodiments, the resolution of the thickness measurement may be affected by the size of the fluorescent beads used. In certain embodiments, the fluorescent beads have an average diameter of about 0.2 µm.

### B. in situ Analysis

In some aspects, the provided embodiments of three-dimensional polymerized matrices and biological samples embedded therein can be employed as samples in an *in situ* method of analyzing nucleic acid sequences, such as *in situ* hybridization and/or *in situ* sequencing, for example from intact tissues or samples in which the spatial information has been preserved. Exemplary *in situ* methods include sequential fluorescent *in situ* hybridization (e.g., MERFISH, SeqFISH, etc.), ligation-based *in situ* sequencing (e.g., Sequencing by Dynamic Annealing and Ligation (SEDAL) as described in WO 2019/199579), and hybridization-based *in situ* sequencing (HybISS) (e.g., as described in WO 2020/240025) or a combination thereof. In some aspects, the embodiments can be applied in an imaging or detection method for multiplexed nucleic acid analysis. In some aspects, the provided embodiments can be used to identify or detect single nucleotides of interest in target nucleic acids. In some aspects, the provided embodiments can be used to crosslink the nucleic acid concatemers via photoreactive nucleotides, e.g., to a complementary strand, to increase the stability of the nucleic acid concatemers probe *in situ.*

In some aspects, the embodiments can be applied in investigative and/or diagnostic applications, for example, for characterization or assessment of particular cell or a tissue from a subject. Applications of the provided method can comprise biomedical research and clinical diagnostics. For example, in biomedical research, applications comprise, but are not limited to, spatially resolved gene expression analysis for biological investigation or drug screening. In clinical diagnostics, applications comprise, but are not limited to, detecting gene markers such as disease, immune responses, bacterial or viral DNA/RNA for patient samples.

In some aspects, the embodiments can be applied to visualize the distribution of genetically encoded markers in whole tissue at subcellular resolution, for example, chromosomal abnormalities (inversions, duplications, translocations, etc.), loss of genetic heterozygosity, the presence of gene alleles indicative of a predisposition towards disease or good health, likelihood of responsiveness to therapy, or in personalized medicine or ancestry.

In some aspects, the provided herein are methods for analyzing, e.g., detecting or determining, the presence of one or more analytes in the biological sample embedded in the three-dimensional polymerized matrix obtained according to the methods described herein. In some aspects, an analyte disclosed herein can include any biological substance, structure, moiety, or component to be analyzed. In some aspects, a target disclosed herein may similarly include any analyte of interest, such as a biomarker.

### (i) Analytes

Systems, apparatus, and methods can be used to analyze any number of analytes present in the biological samples of the present disclosure. For example, the number of analytes that are analyzed can be at least about 2, at least about 3, at least about 4, at least about 5, at least about 6, at least about 7, at least about 8, at least about 9, at least about 10, at least about 11, at least about 12, at least about 13, at least about 14, at least about 15, at least about 20, at least about 25, at least about 30, at least about 40, at least about 50, at least about 100, at least about 1,000, at least about 10,000, at least about 100,000 or more different analytes present in a region of the sample. In some embodiments, each analyte panel comprises 1, 2, 3, 4, 5, 6, 7, 8, 9, 10, or more analytes (e.g., biomarkers). In some embodiments, any one or more of the analyte panels can comprise about 1, about 5, about 10, about 25, about 50, about 100, about 250, about 500, about 1,000, about 2,500, about 5,000 or more analytes (e.g., biomarkers).

Analytes can be derived from a specific type of cell and/or a specific sub-cellular region. For example, analytes can be derived from cytosol, from cell nuclei, from mitochondria, from microsomes, and more generally, from any other compartment, organelle, or portion of a cell. Permeabilizing agents that specifically target certain cell compartments and organelles can be used to selectively release analytes from cells for analysis, and/or allow access of one or more reagents (e.g., probes for *in situ* analysis) to the analytes in the cell or cell compartment or organelle.

Analytes can be broadly classified into one of two groups: nucleic acid analytes, and non-nucleic acid analytes. A method disclosed herein can be used to analyze nucleic acid analytes and/or non-nucleic acid analytes in any suitable combination.

Examples of nucleic acid analytes include DNA analytes such as genomic DNA, methylated DNA, specific methylated DNA sequences, fragmented DNA, mitochondrial DNA, *in situ* synthesized PCR products, and RNA/DNA hybrids.

Examples of nucleic acid analytes also include RNA analytes such as various types of coding and non-coding RNA. Examples of a non-coding RNAs (ncRNA) that is not translated into a protein include transfer RNAs (tRNAs) and ribosomal RNAs (rRNAs), as well as small non-coding RNAs such as microRNA (miRNA), small interfering RNA (siRNA), Piwi-interacting RNA (piRNA), small nucleolar RNA (snoRNA), small nuclear RNA (snRNA), extracellular RNA (exRNA), small Cajal body-specific RNAs (scaRNAs), and the long ncRNAs such as Xist and HOTAIR. Examples of the different types of RNA analytes include messenger RNA (mRNA), including a nascent RNA, a pre-mRNA, a primary-transcript RNA, and a processed RNA, such as a capped mRNA (e.g., with a 5' 7-methyl guanosine cap), a polyadenylated mRNA (poly-A tail at the 3' end), and a spliced mRNA in which one or more introns have been removed. Also included in the analytes disclosed herein are non-capped mRNA, a non-polyadenylated mRNA, and a non-spliced mRNA. Additional examples of RNA analytes include rRNA, tRNA, miRNA, and viral RNA. The RNA can be a transcript (e.g., present in a tissue section). The RNA can be small (e.g., less than 200 nucleic acid bases in length) or large (e.g., RNA greater than 200 nucleic acid bases in length). Examples of small RNAs include 5.8S ribosomal RNA (rRNA), 5S rRNA, tRNA, miRNA, siRNA, snoRNAs, piRNA, tRNA-derived small RNA (tsRNA), and small rDNA-derived RNA (srRNA). The RNA can be double-stranded RNA or single-stranded RNA. The RNA can be circular RNA. The RNA can be a bacterial rRNA (e.g., 16s rRNA or 23s rRNA).

In any of the embodiments herein, the method can comprise analyzing one or more non-nucleic acid analytes, such as protein analytes. In some embodiments, each non-nucleic acid analyte is linked to a labelling agent, e.g., an antibody or antigen binding fragment thereof linked to a reporter oligonucleotide.

Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral coat proteins, extracellular and intracellular proteins, antibodies, and antigen binding fragments. In some embodiments, the analyte is inside a cell or on a cell surface, such as a transmembrane analyte or one that is attached to the cell membrane. In some embodiments, the analyte can be an organelle (e.g., nuclei or mitochondria). In some embodiments, the analyte is an extracellular analyte, such as a secreted analyte.

Cell surface features corresponding to analytes can include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, an extracellular matrix protein, a posttranslational modification (e.g., phosphorylation, glycosylation, ubiquitination, nitrosylation, methylation, acetylation or lipidation) state of a cell surface protein, a gap junction, and an adherens junction.

In certain embodiments, an analyte can be extracted from a live cell. Processing conditions can be adjusted to ensure that a biological sample remains live during analysis, and analytes are extracted from (or released from) live cells of the sample. Live cell-derived analytes can be obtained only once from the sample, or can be obtained at intervals from a sample that continues to remain in viable condition.

In certain embodiments, an analyte or a complex or product thereof may be immobilized in a sample, e.g., to one or more other molecules within the sample and/or within a matrix, generally at the location of the analyte within a native biological sample, e.g., under a physiological or pathological condition and/or while the sample is live. The analyte or a complex or product thereof may be immobilized within the sample and/or matrix by steric factors. The analyte or a complex or product thereof may also be immobilized within the sample and/or matrix by covalent or noncovalent bonding. In this manner, the analyte or a complex or product thereof may be considered to be attached to the sample or matrix. By being immobilized to the sample and/or matrix, such as by covalent bonding or cross-linking, the size and/or spatial relationship of the analyte or a complex or product thereof can be maintained. By being immobilized to the sample and/or matrix, such as by covalent bonding or cross-linking, the analyte or a complex or product thereof is resistant to movement or unraveling under mechanical stress.

### (ii) Labelling Agents

In some embodiments, an analyte labelling agent (also referred to at times as a "capture agent") may include an agent that interacts with an analyte (e.g., an analyte in a sample) and with a probe to identify the analyte. In some embodiments, the sample may be contacted with one or more labelling agents prior to, during, or after an *in situ* assay. In some embodiments, the method comprises one or more post-fixing (also referred to as post-fixation) steps after contacting the sample with one or more labelling agents. In some embodiments, the analyte labelling agent comprises an analyte binding moiety and a labelling agent barcode domain (e.g., an analyte binding moiety barcode).

In the methods and systems described herein, one or more labelling agents capable of binding to or otherwise coupling to one or more features may be used to characterize analytes, cells and/or cell features. In some instances, cell features include cell surface features. Analytes may include, but are not limited to, a protein, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. In some instances, cell features may include intracellular analytes, such as proteins, protein modifications (e.g., phosphorylation status or other post-translational modifications), nuclear proteins, nuclear membrane proteins, or any combination thereof.

A labelling agent may include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include (e.g., are attached to) a reporter oligonucleotide that is indicative of the cell surface feature to which the binding group binds. For example, the reporter oligonucleotide may comprise a barcode sequence that permits identification of the labelling agent. For example, a labelling agent that is specific to one type of cell feature (e.g., a first cell surface feature) may have coupled thereto a first reporter oligonucleotide, while a labelling agent that is specific to a different cell feature (e.g., a second cell surface feature) may have a different reporter oligonucleotide coupled thereto. For a description of exemplary labelling agents, reporter oligonucleotides, and methods of use, see, e.g., U.S. Pat. 10,550,429; U.S. Pat. Pub. 20190177800; and U.S. Pat. Pub. 20190367969, which are each incorporated by reference herein in their entirety.

In other instances, e.g., to facilitate sample multiplexing, a labelling agent that is specific to a particular cell feature may have a first plurality of the labelling agent (e.g., an antibody or lipophilic moiety) coupled to a first reporter oligonucleotide and a second plurality of the labelling agent coupled to a second reporter oligonucleotide.

In some aspects, these reporter oligonucleotides may comprise nucleic acid barcode sequences that permit identification of the labelling agent which the reporter oligonucleotide is coupled to. The selection of oligonucleotides as the reporter may provide advantages of being able to generate significant diversity in terms of sequence, while also being readily attachable to most biomolecules, e.g., antibodies, etc., as well as being readily detected, e.g., using sequencing or array technologies.

Attachment (coupling) of the reporter oligonucleotides to the labelling agents may be achieved through any of a variety of direct or indirect, covalent or non-covalent associations or attachments. For example, oligonucleotides may be covalently attached to a portion of a labelling agent (such a protein, e.g., an antibody or antibody fragment) using chemical conjugation techniques (e.g., Lightning-Link^{®} antibody labelling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715, which is entirely incorporated herein by reference for all purposes. Likewise, protein and peptide biotinylation techniques have been developed and are readily available. See, e.g., U.S. Pat. No. 6,265,552, which is entirely incorporated herein by reference for all purposes. Furthermore, click reaction chemistry such as a Methyltetrazine-PEG5-NHS Ester reaction, a TCO-PEG4-NHS Ester reaction, or the like, may be used to couple reporter oligonucleotides to labelling agents. Commercially available kits, such as those from Thunderlink and Abcam, and techniques common in the art may be used to couple reporter oligonucleotides to labelling agents as appropriate. In another example, a labelling agent is indirectly (e.g., via hybridization) coupled to a reporter oligonucleotide comprising a barcode sequence that identifies the label agent. For instance, the labelling agent may be directly coupled (e.g., covalently bound) to a hybridization oligonucleotide that comprises a sequence that hybridizes with a sequence of the reporter oligonucleotide. Hybridization of the hybridization oligonucleotide to the reporter oligonucleotide couples the labelling agent to the reporter oligonucleotide. In some embodiments, the reporter oligonucleotides are releasable from the labelling agent, such as upon application of a stimulus. For example, the reporter oligonucleotide may be attached to the labeling agent through a labile bond (e.g., chemically labile, photolabile, thermally labile, etc.) as generally described for releasing molecules from supports elsewhere herein. In some instances, the reporter oligonucleotides described herein may include one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, a sequencing primer or primer biding sequence (such as an R1, R2, or partial R1 or R2 sequence).

In some cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (e.g., the label can be conjugated to a molecule that can bind to the labelling agent or reporter oligonucleotide). In some cases, a label is conjugated to a first oligonucleotide that is complementary (e.g., hybridizes) to a sequence of the reporter oligonucleotide.

In some embodiments, an analyte binding moiety may include any molecule or moiety capable of binding to an analyte (e.g., a biological analyte, e.g., a macromolecular constituent). In some embodiments, disclosed herein is a method wherein the analyte binding moiety that binds to a biological analyte can include, but is not limited to, an antibody, or an epitope binding fragment thereof, a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The analyte binding moiety can bind to the macromolecular constituent (e.g., analyte) with high affinity and/or with high specificity. The analyte binding moiety can include a nucleotide sequence (e.g., an oligonucleotide), which can correspond to at least a portion or an entirety of the analyte binding moiety. The analyte binding moiety can include a polypeptide and/or an aptamer (e.g., a polypeptide and/or an aptamer that binds to a specific target molecule, e.g., an analyte). The analyte binding moiety can include an antibody or antibody fragment (e.g., an antigen-binding fragment) that binds to a specific analyte (e.g., a polypeptide).

As used herein, the term "analyte binding moiety barcode" refers to a barcode that is associated with or otherwise identifies the analyte binding moiety. In some embodiments, by identifying an analyte binding moiety by identifying its associated analyte binding moiety barcode, the analyte to which the analyte binding moiety binds can also be identified. An analyte binding moiety barcode can be a nucleic acid sequence of a given length and/or sequence that is associated with the analyte binding moiety. An analyte binding moiety barcode can generally include any of the variety of aspects of barcodes described herein.

In some embodiments, an analyte binding moiety includes one or more antibodies or antigen binding fragments thereof. The antibodies or antigen binding fragments including the analyte binding moiety can specifically bind to a target analyte. In some embodiments, the analyte is a protein (e.g., a protein on a surface of the biological sample (e.g., a cell) or an intracellular protein). In some embodiments, a plurality of analyte labelling agents comprising a plurality of analyte binding moieties bind a plurality of analytes present in a biological sample. In some embodiments, the plurality of analytes includes a single species of analyte (e.g., a single species of polypeptide). In some embodiments in which the plurality of analytes includes a single species of analyte, the analyte binding moieties of the plurality of analyte labelling agents are the same. In some embodiments in which the plurality of analytes includes a single species of analyte, the analyte binding moieties of the plurality of analyte labelling agents are the different (e.g., members of the plurality of analyte labelling agents can have two or more species of analyte binding moieties, wherein each of the two or more species of analyte binding moieties binds a single species of analyte, e.g., at different binding sites). In some embodiments, the plurality of analytes includes multiple different species of analyte (e.g., multiple different species of polypeptides).

In some embodiments, multiple different species of analytes (e.g., polypeptides) from the biological sample can be subsequently associated with the one or more physical properties of the biological sample. For example, the multiple different species of analytes can be associated with locations of the analytes in the biological sample. Such information (e.g., proteomic information when the analyte binding moiety(ies) recognizes a polypeptide(s)) can be used in association with other spatial information (e.g., genetic information from the biological sample, such as DNA sequence information, transcriptome information (i.e., sequences of transcripts), or both). For example, a cell surface protein of a cell can be associated with one or more physical properties of the cell (e.g., a shape, size, activity, or a type of the cell). The one or more physical properties can be characterized by imaging the cell. The cell can be bound by an analyte labelling agent comprising an analyte binding moiety that binds to the cell surface protein and an analyte binding moiety barcode that identifies that analyte binding moiety, and the cell can be subjected to spatial analysis (e.g., any of the variety of spatial analysis methods described herein). Non-limiting aspects of spatial analysis methodologies are described in U.S. Pat. Pub. No. 10,308,982; U.S. Pat. Pub. No. 9,879,313; U.S. Pat. Pub. No. 9,868,979; Liu et al., bioRxiv 788992, 2020; U.S. Pat. Pub. No. 10,774,372; U.S. Pat. Pub. No. 10,774,374; WO 2018/091676; U.S. Pat. Pub. No. 10,030,261; U.S. Pat. Pub. No. 9,593,365; U.S. Patent No. 10,002,316; U.S. Patent No. 9,727,810; U.S. Pat. Pub. No. 10,640,816; Rodriques et al., Science 363(6434):1463-1467, 2019; Lee et al., Nat. Protoc. 10(3):442-458, 2015; U.S. Pat. Pub. No. 10,179,932; U.S. Pat. Pub. No. 10,138,509; Trejo et al., PLoS ONE 14(2):e0212031, 2019; U.S. Patent Application Publication Nos. 2018/0245142; 2019/0177718; Chen et al., Science 348(6233):aaa6090, 2015; Gao et al., BMC Biol. 15:50, 2017; WO 2018/107054; U.S. Patent Application Publication Nos. 2019/0161796; 2020/0224244; 2019/0194709; WO 2011/094669; U.S. Patent No. 7,709,198; U.S. Patent No. 8,604,182; U.S. Patent No. 8,951,726; U.S. Patent No. 9,783,841; U.S. Patent No. 10,041,949; WO 2016/057552; WO 2017/147483; U.S. Pat. Pub. Nos. 10,370,698; 10,724,078; 10,364,457; U.S. Pat. Pub. No. 10,317,321; WO 2018/136856; U.S. Patent Application Publication Nos. 2017/0241911; 2017/0029875; U.S. Patent No. 10,059,990; WO 2018/057999; and Gupta et al., Nature Biotechnol. 36:1197-1202, 2018, and can be used herein in any combination. Further non-limiting aspects of spatial analysis methodologies are described herein.

### (iii) Signal Amplification and/or Detection

In some aspects, a three-dimensional polymerized matrix disclosed herein and/or a biological sample embedded in the three-dimensional polymerized matrix may be subjected to *in situ* analysis, including *in situ* sequencing and/or *in situ* hybridization-based analysis of an intact tissue or non-homogenized tissue.

### a. in situ Signal Amplification

In some aspects, the provided methods involve analyzing, e.g., detecting or determining, one or more sequences (e.g., any of the barcodes described herein) and/or in a product or derivative thereof. In some embodiments, a method disclosed herein may also comprise one or more *in situ* signal amplification components. In some embodiments, the present disclosure relates to the detection of nucleic acids sequences *in situ* using probe hybridization and generation of amplified signals associated with the probes, wherein background signal is reduced and sensitivity is increased.

Exemplary signal amplification methods include targeted deposition of detectable reactive molecules around the site of probe hybridization, targeted assembly of branched structures (e.g., bDNA or branched assay using locked nucleic acid (LNA)), programmed *in situ* growth of concatemers by enzymatic rolling circle amplification (RCA) (e.g., as described in US 2019/0055594 incorporated herein by reference), hybridization chain reaction, assembly of topologically catenated DNA structures using serial rounds of chemical ligation (clampFISH), signal amplification via hairpin-mediated concatemerization (e.g., as described in US 2020/0362398 incorporated herein by reference), e.g., primer exchange reactions such as signal amplification by exchange reaction (SABER) or SABER with DNA-Exchange (Exchange-SABER), or any combination thereof. In some embodiments, non-enzymatic signal amplification may be used.

The detectable reactive molecules may comprise tyramide, such as used in tyramide signal amplification (TSA) or multiplexed catalyzed reporter deposition (CARD)-FISH. In some embodiments, the detectable reactive molecule may be releasable and/or cleavable from a detectable label such as a fluorophore. In some embodiments, a method disclosed herein comprises multiplexed analysis of a biological sample comprising consecutive cycles of probe hybridization, fluorescence imaging, and signal removal, where the signal removal comprises removing the fluorophore from a fluorophore-labeled reactive molecule (e.g., tyramide). Exemplary detectable reactive reagents and methods are described in US 6,828,109, US 2019/0376956, WO 2019/236841, WO 2020/102094, WO 2020/163397, and WO 2021/067475, all of which are incorporated herein by reference in their entireties.

In some embodiments, hybridization chain reaction (HCR) can be used for signal amplification. HCR is an enzyme-free nucleic acid amplification based on a triggered chain of hybridization of nucleic acid molecules starting from HCR monomers, which hybridize to one another to form a nicked nucleic acid polymer. This polymer is the product of the HCR reaction which is ultimately detected in order to indicate the presence of the target analyte. HCR is described in detail in Dirks and Pierce, 2004, PNAS, 101(43), 15275-15278 and in US 7,632,641 and US 7,721,721 (see also US 2006/00234261; Chemeris et al, 2008 Doklady Biochemistry and Biophysics, 419, 53-55; Niu et al, 2010, 46, 3089-3091; Choi et al, 2010, Nat. Biotechnol. 28(11), 1208-1212; and Song et al, 2012, Analyst, 137, 1396-1401). HCR monomers typically comprise a hairpin, or other metastable nucleic acid structure. In the simplest form of HCR, two different types of stable hairpin monomer, referred to here as first and second HCR monomers, undergo a chain reaction of hybridization events to form a long nicked double-stranded DNA molecule when an "initiator" nucleic acid molecule is introduced. The HCR monomers have a hairpin structure comprising a double stranded stem region, a loop region connecting the two strands of the stem region, and a single stranded region at one end of the double stranded stem region. The single stranded region which is exposed (and which is thus available for hybridization to another molecule, e.g. initiator or other HCR monomer) when the monomers are in the hairpin structure may be known as the "toehold region" (or "input domain"). The first HCR monomers each further comprise a sequence which is complementary to a sequence in the exposed toehold region of the second HCR monomers. This sequence of complementarity in the first HCR monomers may be known as the "interacting region" (or "output domain"). Similarly, the second HCR monomers each comprise an interacting region (output domain), e.g. a sequence which is complementary to the exposed toehold region (input domain) of the first HCR monomers. In the absence of the HCR initiator, these interacting regions are protected by the secondary structure (e.g. they are not exposed), and thus the hairpin monomers are stable or kinetically trapped (also referred to as "metastable"), and remain as monomers (e.g. preventing the system from rapidly equilibrating), because the first and second sets of HCR monomers cannot hybridize to each other. However, once the initiator is introduced, it is able to hybridize to the exposed toehold region of a first HCR monomer, and invade it, causing it to open up. This exposes the interacting region of the first HCR monomer (e.g. the sequence of complementarity to the toehold region of the second HCR monomers), allowing it to hybridize to and invade a second HCR monomer at the toehold region. This hybridization and invasion in turn opens up the second HCR monomer, exposing its interacting region (which is complementary to the toehold region of the first HCR monomers), and allowing it to hybridize to and invade another first HCR monomer. The reaction continues in this manner until all of the HCR monomers are exhausted (e.g. all of the HCR monomers are incorporated into a polymeric chain). Ultimately, this chain reaction leads to the formation of a nicked chain of alternating units of the first and second monomer species. The presence of the HCR initiator is thus required in order to trigger the HCR reaction by hybridization to and invasion of a first HCR monomer. The first and second HCR monomers are designed to hybridize to one another are thus may be defined as cognate to one another. They are also cognate to a given HCR initiator sequence. HCR monomers which interact with one another (hybridize) may be described as a set of HCR monomers or an HCR monomer, or hairpin, system.

An HCR reaction could be carried out with more than two species or types of HCR monomers. For example, a system involving three HCR monomers could be used. In such a system, each first HCR monomer may comprise an interacting region which binds to the toehold region of a second HCR monomer; each second HCR may comprise an interacting region which binds to the toehold region of a third HCR monomer; and each third HCR monomer may comprise an interacting region which binds to the toehold region of a first HCR monomer. The HCR polymerization reaction would then proceed as described above, except that the resulting product would be a polymer having a repeating unit of first, second and third monomers consecutively. Corresponding systems with larger numbers of sets of HCR monomers could readily be conceived. Branching HCR systems have also been devised and described (see, e.g., WO 2020/123742 incorporated herein by reference), and may be used in the methods herein.

In some embodiments, similar to HCR reactions that use hairpin monomers, linear oligo hybridization chain reaction (LO-HCR) can also be used for signal amplification. In some embodiments, provided herein is a method of detecting an analyte in a sample comprising: (i) performing a linear oligo hybridization chain reaction (LO-HCR), wherein an initiator is contacted with a plurality of LO-HCR monomers of at least a first and a second species to generate a polymeric LO-HCR product hybridized to a target nucleic acid molecule, wherein the first species comprises a first hybridization region complementary to the initiator and a second hybridization region complementary to the second species, wherein the first species and the second species are linear, single-stranded nucleic acid molecules; wherein the initiator is provided in one or more parts, and hybridizes directly or indirectly to or is comprised in the target nucleic acid molecule; and (ii) detecting the polymeric product, thereby detecting the analyte. In some embodiments, the first species and/or the second species may not comprise a hairpin structure. In some embodiments, the plurality of LO-HCR monomers may not comprise a metastable secondary structure. In some embodiments, the LO-HCR polymer may not comprise a branched structure. In some embodiments, performing the linear oligo hybridization chain reaction comprises contacting the target nucleic acid molecule with the initiator to provide the initiator hybridized to the target nucleic acid molecule. In any of the embodiments herein, the target nucleic acid molecule and/or the analyte can be an RCA product.

In some embodiments, detection of nucleic acids sequences *in situ* includes an assembly for branched signal amplification. In some embodiments, the assembly complex comprises an amplifier hybridized directly or indirectly (via one or more oligonucleotides) to a sequence (e.g., any of the barcodes described herein) present in a probe described herein and/or in a product or derivative thereof. In some embodiments, the assembly includes one or more amplifiers each including an amplifier repeating sequence. In some aspects, the one or more amplifiers is labeled. Described herein is a method of using the aforementioned assembly, including for example, using the assembly in multiplexed error-robust fluorescent in situ hybridization (MERFISH) applications, with branched DNA amplification for signal readout. In some embodiments, the amplifier repeating sequence is about 5-30 nucleotides, and is repeated N times in the amplifier. In some embodiments, the amplifier repeating sequence is about 20 nucleotides, and is repeated at least two times in the amplifier. In some aspects, the one or more amplifier repeating sequence is labeled. For exemplary branched signal amplification, see e.g., U.S. Pat. Pub. No. US20200399689A1 and Xia et al., Multiplexed Detection of RNA using MERFISH and branched DNA amplification, Scientific Reports (2019), each of which is fully incorporated by reference herein.

In some embodiments, the sequences (e.g., any of the barcodes described herein) present in a probe described herein and/or in a product or derivative thereof can be detected in with a method that comprises signal amplification by performing a primer exchange reaction (PER). In various embodiments, a primer with domain on its 3' end binds to a catalytic hairpin, and is extended with a new domain by a strand displacing polymerase. For example, a primer with domain 1 on its 3' ends binds to a catalytic hairpin, and is extended with a new domain 1 by a strand displacing polymerase, with repeated cycles generating a concatemer of repeated domain 1 sequences. In various embodiments, the strand displacing polymerase is Bst. In various embodiments, the catalytic hairpin includes a stopper which releases the strand displacing polymerase. In various embodiments, branch migration displaces the extended primer, which can then dissociate. In various embodiments, the primer undergoes repeated cycles to form a concatemer primer. In various embodiments, the sample may be contacted with a plurality of concatemer primers and a plurality of labeled probes. see e.g., U.S. Pat. Pub. No. US20190106733, which is incorporated herein by reference, for exemplary molecules and PER reaction components.

In some embodiments, a product of an endogenous analyte and/or a labelling agent is an amplification product of one or more polynucleotides, for instance, a circular probe or circularizable probe or probe set. In some embodiments, the amplifying is achieved by performing rolling circle amplification (RCA). In other embodiments, a primer that hybridizes to the circular probe or circularized probe is added and used as such for amplification. In some embodiments, the RCA comprises a linear RCA, a branched RCA, a dendritic RCA, or any combination thereof.

In some embodiments, the amplification is performed at a temperature between or between about 20°C and about 60°C. In some embodiments, the amplification is performed at a temperature between or between about 30°C and about 40°C. In some aspects, the amplification step, such as the rolling circle amplification (RCA) is performed at a temperature between at or about 25°C and at or about 50°C, such as at or about 25°C, 27°C, 29°C, 31°C, 33°C, 35°C, 37°C, 39°C, 41°C, 43°C, 45°C, 47°C, or 49°C.

In some embodiments, upon addition of a DNA polymerase in the presence of appropriate dNTP precursors and other cofactors, a primer is elongated to produce multiple copies of the circular template. This amplification step can utilize isothermal amplification or non-isothermal amplification. In some embodiments, after the formation of the hybridization complex and association of the amplification probe, the hybridization complex is rolling-circle amplified to generate an amplicon (e.g., a DNA nanoball) containing multiple copies of the circular template or a sequence thereof. Techniques for rolling circle amplification (RCA) include linear RCA, a branched RCA, a dendritic RCA, or any combination thereof. (See, e.g., Baner et al, Nucleic Acids Research, 26:5073-5078, 1998; Lizardi et al, Nature Genetics 19:226, 1998; Mohsen et al., Acc Chem Res. 2016 November 15; 49(11): 2540-2550; Schweitzer et al. Proc. Natl Acad. Sci. USA 97:101 13- 1 19, 2000; Faruqi et al, BMC Genomics 2:4, 2000; Nallur et al, Nucl. Acids Res. 29:el 18, 2001; Dean et al. Genome Res. 1 1 :l095- 1099, 2001; Schweitzer et al, Nature Biotech. 20:359-365, 2002; U.S. Patent Nos. 6,054,274, 6,291,187, 6,323,009, 6,344,329 and 6,368,801). Exemplary polymerases for use in RCA comprise DNA polymerase such phi29 (φ29) polymerase, Klenow fragment, *Bacillus stearothermophilus* DNA polymerase (BST), T4 DNA polymerase, T7 DNA polymerase, or DNA polymerase I. In some aspects, DNA polymerases that have been engineered or mutated to have desirable characteristics can be employed. In some embodiments, the polymerase is phi29 DNA polymerase.

In some aspects, during the amplification step, modified nucleotides can be added to the reaction to incorporate the modified nucleotides in the amplification product (e.g., nanoball). Exemplary of the modified nucleotides comprise amine-modified nucleotides. In some aspects of the methods, for example, for anchoring or cross-linking of the generated amplification product (e.g., nanoball) to a scaffold, to cellular structures and/or to other amplification products (e.g., other nanoballs). In some aspects, the amplification products comprises a modified nucleotide, such as an amine-modified nucleotide. In some embodiments, the amine-modified nucleotide comprises an acrylic acid N-hydroxysuccinimide moiety modification. Examples of other amine-modified nucleotides comprise, but are not limited to, a 5-Aminoallyl-dUTP moiety modification, a 5-Propargylamino-dCTP moiety modification, a N6-6-Aminohexyl-dATP moiety modification, or a 7-Deaza-7-Propargylamino-dATP moiety modification.

In some aspects, the polynucleotides and/or amplification product (e.g., amplicon) can be anchored to a polymer matrix. For example, the polymer matrix can be a hydrogel. In some embodiments, one or more of the polynucleotide probe(s) can be modified to contain functional groups that can be used as an anchoring site to attach the polynucleotide probes and/or amplification product to a polymer matrix. Exemplary modification and polymer matrix that can be employed in accordance with the provided embodiments comprise those described in, for example, US 10,138,509, US 10,266,888, US 10,494,662, and US 10,545,075, all of which are incorporated herein by reference. In some examples, the scaffold also contains modifications or functional groups that can react with or incorporate the modifications or functional groups of the probe set or amplification product. In some examples, the scaffold can comprise oligonucleotides, polymers or chemical groups, to provide a matrix and/or support structures.

The amplification products may be immobilized within the matrix generally at the location of the nucleic acid being amplified, thereby creating a localized colony of amplicons. The amplification products may be immobilized within the matrix by steric factors. The amplification products may also be immobilized within the matrix by covalent or noncovalent bonding. In this manner, the amplification products may be considered to be attached to the matrix. By being immobilized to the matrix, such as by covalent bonding or cross-linking, the size and spatial relationship of the original amplicons is maintained. By being immobilized to the matrix, such as by covalent bonding or cross-linking, the amplification products are resistant to movement or unraveling under mechanical stress.

In some aspects, the amplification products are copolymerized and/or covalently attached to the surrounding matrix thereby preserving their spatial relationship and any information inherent thereto. For example, if the amplification products are those generated from DNA or RNA within a cell embedded in the matrix, the amplification products can also be functionalized to form covalent attachment to the matrix preserving their spatial information within the cell thereby providing a subcellular localization distribution pattern. In some embodiments, the provided methods involve embedding the one or more polynucleotide probe sets and/or the amplification products in the presence of hydrogel subunits to form one or more hydrogel-embedded amplification products. In some embodiments, the hydrogel-tissue chemistry described comprises covalently attaching nucleic acids to *in situ* synthesized hydrogel for tissue clearing, enzyme diffusion, and multiple-cycle sequencing while an existing hydrogel-tissue chemistry method cannot. In some embodiments, to enable amplification product embedding in the tissue-hydrogel setting, amine-modified nucleotides are comprised in the amplification step (e.g., RCA), functionalized with an acrylamide moiety using acrylic acid N-hydroxysuccinimide esters, and copolymerized with acrylamide monomers to form a hydrogel.

### b. Signal Detection

In some embodiments, provided herein are methods comprising *in situ* assays using microscopy as a readout, e.g., nucleic acid sequencing, hybridization, or other detection or determination methods involving an optical readout. In some cases, analysis is performed on one or more images captured, and may comprise processing the image(s) and/or quantifying signals observed. In some embodiments, images of signals from different fluorescent channels and/or detectable probe hybridization (and optionally ligation) cycles can be compared and analyzed. In some embodiments, images of signals (or absence thereof) at a particular location in a sample from different fluorescent channels and/or sequential detectable probe hybridization (and optionally ligation) cycles can be aligned to analyze an analyte at the location. For instance, a particular location in a sample can be tracked and signal spots from sequential hybridization (and optionally ligation) cycles can be analyzed to detect a target polynucleotide sequence (e.g., a barcode sequence or subsequence thereof) in a nucleic acid at the location. The analysis may comprise processing information of one or more cell types, one or more types of analytes, a number or level of analyte, and/or a number or level of cells detected in a particular region of the sample. In some embodiments, the analysis comprises detecting a sequence e.g., a barcode sequence present in an amplification product at a location in the sample. In some embodiments, the analysis includes quantification of puncta (e.g., if amplification products are detected). In some cases, the analysis includes determining whether particular cells and/or signals are present that correlate with one or more analytes from a particular panel. In some embodiments, the obtained information may be compared to a positive and negative control, or to a threshold of a feature to determine if the sample exhibits a certain feature or phenotype. In some cases, the information may comprise signals from a cell, a region, and/or comprise readouts from multiple detectable labels. In some case, the analysis further includes displaying the information from the analysis or detection step. In some embodiments, software may be used to automate the processing, analysis, and/or display of data.

In some aspects, detection or determination of a sequence of one, two, three, four, five, or more nucleotides of a target nucleic acid is performed *in situ* in a cell in an intact tissue. In some embodiments, the assay comprises detecting the presence or absence of an amplification product (e.g., RCA product). In some embodiments, the present disclosure provides methods for high-throughput profiling of a large number of targets *in situ,* such as transcripts and/or DNA loci, e.g., for detecting and/or quantifying nucleic acids and/or proteins in cells, tissues, organs or organisms.

In some aspects, provided herein is a method comprising analyzing biological targets based on *in situ* hybridization of probes comprising nucleic acid sequences. In some embodiments, the method comprises sequential hybridization of detectably-labelled oligonucleotides to barcoded probes that directly or indirectly bind to biological targets in a sample. In some embodiments, a detectably-labelled oligonucleotide directly binds to one or more barcoded probes. In some embodiments, a detectably-labelled oligonucleotide indirectly binds to one or more barcoded probes, e.g., via one or more bridging nucleic acid molecules.

In some aspects, an *in situ* hybridization based assay is used to localize and analyze nucleic acid sequences (e.g., a DNA or RNA molecule comprising one or more specific sequences of interest) within a native biological sample, e.g., a portion or section of tissue or a single cell. In some embodiments, the *in situ* assay is used to analyze the presence, absence, an amount or level of mRNA transcripts (e.g., a transcriptome or a subset thereof, or mRNA molecules of interest) in a biological sample, while preserving spatial context. In some embodiments, the present disclosure provides compositions and methods for *in situ* hybridization using directly or indirectly labeled molecules, e.g., complementary DNA or RNA or modified nucleic acids, as probes that bind or hybridize to a target nucleic acids within a biological sample of interest.

Nucleic acid probes, in some examples, may be labelled with radioisotopes, epitopes, hapten, biotin, or fluorophores, to enable detection of the location of specific nucleic acid sequences on chromosomes or in tissues. In some embodiments, probes are locus specific (e.g., gene specific) and bind or couple to specific regions of a chromosome. In alternative embodiments, probes are alphoid or centromeric repeat probes that bind or couple to repetitive sequences within each chromosome. Probes may also be whole chromosome probes (e.g., multiple smaller probes) that bind or couple to sequences along an entire chromosome.

In some embodiments, provided herein is a method comprising DNA *in situ* hybridization to measure and localize DNA. In some embodiments, provided herein is a method RNA *in situ* hybridization to measure and localize RNAs (e.g., mRNAs, lncRNAs, and miRNAs) within a biological sample (e.g., a fixed tissue sample). In some embodiments, RNA *in situ* hybridization involves single-molecule RNA fluorescence *in situ* hybridization (FISH). In some embodiments, fluorescently labelled nucleic acid probes are hybridized to pre-determined RNA targets, to visualize gene expression in a biological sample. In some embodiments, a FISH method comprises using a single nucleic acid probe specific to each target, e.g., single-molecule FISH (smFISH). The use of smFISH may produce a fluorescence signal that allows for quantitative measurement of RNA transcripts. In some embodiments, smFISH comprises a set of nucleic acid probes, about 50 base pairs in length, wherein each probe is coupled to a set fluorophores. For example, the set of nucleic acid probes may comprise five probes, wherein each probe coupled to five fluorophores. In some embodiments, said nucleic acid probes are instead each coupled to one fluorophore. For example, a smFISH protocol may use a set of about 40 nucleic acid probes, about 20 base pairs in length, each coupled to a single fluorophore. In some embodiments, the length of the nucleic acid probes varies, comprising 10 to 100 base pairs, such as 30 to 60 base pairs. Alternatively, a plurality of nucleic acid probes targeting different regions of the same RNA transcript may be used. It will be appreciated by those skilled in the art that the type of nucleic acid probes, the number of nucleic acid probes, the number of fluorophores coupled to said probes, and the length of said probes, may be varied to fit the specifications of the individual assay.

In some aspects, the provided methods further involve analyzing, e.g., detecting or determining, one or more sequences present in the target nucleic acid and/or in the nucleic acid as described herein. In some embodiments, the detecting comprises hybridizing one or more detectably labeled probes to the nucleic acid concatemer, or via hybridization to adaptor probes that hybridize to the nucleic acid concatemer). In some embodiments, the analysis comprises determining the sequence of all or a portion of the nucleic acid concatemer (e.g., a barcode sequence or a complement thereof), wherein the sequence is indicative of a sequence of the target nucleic acid.

Methods for binding and identifying a target nucleic acid that uses various probes or oligonucleotides have been described in, e.g., US2003/0013091, US2007/0166708, US2010/0015607, US2010/0261026, US2010/0262374, US2010/0112710, US2010/0047924, and US2014/0371088, each of which is incorporated herein by reference in its entirety. Detectably-labeled probes can be useful for detecting multiple target nucleic acids and be detected in one or more hybridization cycles (e.g., sequential hybridization in a FISH-type assay, sequencing by hybridization).

In some embodiments, the methods comprise sequencing all or a portion of the nucleic acid. In some embodiments, the sequence of the nucleic acid, or barcode thereof, is indicative of a sequence of the target nucleic acid to which the nucleic acid is hybridized. In some embodiments, the analysis and/or sequence determination comprises sequencing all or a portion of the nucleic acid and/or *in situ* hybridization to the nucleic acid. In some embodiments, the sequencing step involves sequencing by hybridization, sequencing by ligation, sequencing by synthesis, sequencing by binding, and/or fluorescent *in situ* sequencing (FISSEQ), hybridization-based *in situ* sequencing and/or wherein the *in situ* hybridization comprises sequential fluorescent *in situ* hybridization. In some embodiments, the analysis and/or sequence determination comprises detecting a polymer generated by a hybridization chain reaction (HCR) reaction, see e.g., US2017/0009278, which is incorporated herein by reference, for exemplary probes and HCR reaction components. In some embodiments, the detection or determination comprises hybridizing to the first overhang a detection oligonucleotide labeled with a fluorophore, an isotope, a mass tag, or a combination thereof. In some embodiments, the detection or determination comprises imaging the probe hybridized to the target nucleic acid (e.g., imaging one or more detectably labeled probes hybridized thereto). In some embodiments, the target nucleic acid is an mRNA in a tissue sample, and the detection or determination is performed when the target nucleic acid and/or the amplification product is *in situ* in the tissue sample. In some embodiments, the target nucleic acid is an amplification product (e.g., a rolling circle amplification product/nucleic acid).

In some aspects, the provided methods comprise imaging the probe hybridized to the nucleic acid, for example, via binding of one or more detectable probes and detecting signals associated with detectable labels. In some embodiments, the detectable probe comprises a detectable label that can be measured and quantitated. The terms "label" and "detectable label" comprise a directly or indirectly detectable moiety that is associated with (e.g., conjugated to) a molecule to be detected, comprising, but not limited to, fluorophores, radioactive isotopes, fluorescers, chemiluminescers, enzymes, enzyme substrates, enzyme cofactors, enzyme inhibitors, chromophores, dyes, metal ions, metal sols, ligands (e.g., biotin or haptens) and the like.

The term "fluorophore" comprises a substance or a portion thereof that is capable of exhibiting fluorescence in the detectable range. Particular examples of labels that may be used in accordance with the provided embodiments comprise, but are not limited to phycoerythrin, Alexa dyes, fluorescein, YPet, CyPet, Cascade blue, allophycocyanin, Cy3, Cy5, Cy7, rhodamine, dansyl, umbelliferone, Texas red, luminol, acradimum esters, biotin, green fluorescent protein (GFP), enhanced green fluorescent protein (EGFP), yellow fluorescent protein (YFP), enhanced yellow fluorescent protein (EYFP), blue fluorescent protein (BFP), red fluorescent protein (RFP), firefly luciferase, Renilla luciferase, NADPH, beta-galactosidase, horseradish peroxidase, glucose oxidase, alkaline phosphatase, chloramphenical acetyl transferase, and urease.

Fluorescence detection in tissue samples can often be hindered by the presence of strong background fluorescence. "Autofluorescence" is the general term used to distinguish background fluorescence (that can arise from a variety of sources, including aldehyde fixation, extracellular matrix components, red blood cells, lipofuscin, and the like) from the desired immunofluorescence from the fluorescently labeled antibodies or probes. Tissue autofluorescence can lead to difficulties in distinguishing the signals due to fluorescent antibodies or probes from the general background. In some embodiments, a method disclosed herein utilizes one or more agents to reduce tissue autofluorescence, for example, Autofluorescence Eliminator (Sigma/EMD Millipore), TrueBlack Lipofuscin Autofluorescence Quencher (Biotium), MaxBlock Autofluorescence Reducing Reagent Kit (MaxVision Biosciences), and/or a very intense black dye (e.g., Sudan Black, or comparable dark chromophore).

In some embodiments, a detectable probe containing a detectable label can be used to detect one or more nucleic acid concatemer(s) crosslinked to the complementary strand of the target nucleic acid described herein. In some embodiments, the methods involve incubating the detectable probe containing the detectable label with the sample, washing unbound detectable probe, and detecting the label, e.g., by imaging. In some embodiments, the nucleic acid concatemer(s) remain crosslinked to the target nucleic acid during the washing and detecting steps.

Examples of detectable labels comprise but are not limited to various radioactive moieties, enzymes, prosthetic groups, fluorescent markers, luminescent markers, bioluminescent markers, metal particles, protein-protein binding pairs and protein-antibody binding pairs. Examples of fluorescent proteins comprise, but are not limited to, yellow fluorescent protein (YFP), green fluorescence protein (GFP), cyan fluorescence protein (CFP), umbelliferone, fluorescein, fluorescein isothiocyanate, rhodamine, dichlorotriazinylamine fluorescein, dansyl chloride and phycoerythrin.

Examples of bioluminescent markers comprise, but are not limited to, luciferase (e.g., bacterial, firefly and click beetle), luciferin, aequorin and the like. Examples of enzyme systems having visually detectable signals comprise, but are not limited to, galactosidases, glucorimidases, phosphatases, peroxidases and cholinesterases. Identifiable markers also comprise radioactive compounds such as ¹²⁵I, ³⁵S, ¹⁴C, or ³H. Identifiable markers are commercially available from a variety of sources.

Examples of fluorescent labels and nucleotides and/or polynucleotides conjugated to such fluorescent labels comprise those described in, for example, Hoagland, Handbook of Fluorescent Probes and Research Chemicals, Ninth Edition (Molecular Probes, Inc., Eugene, 2002); Keller and Manak, DNA Probes, 2nd Edition (Stockton Press, New York, 1993); Eckstein, editor, Oligonucleotides and Analogues: A Practical Approach (IRL Press, Oxford, 1991); and Wetmur, Critical Reviews in Biochemistry and Molecular Biology, 26:227- 259

(1991). In some embodiments, exemplary techniques and methods methodologies applicable to the provided embodiments comprise those described in, for example, US 4,757,141, US 5,151,507 and US 5,091,519. In some embodiments, one or more fluorescent dyes are used as labels for labeled target sequences, for example, as described in US 5,188,934 (4,7-dichlorofluorescein dyes); US 5,366,860 (spectrally resolvable rhodamine dyes); US 5,847,162 (4,7- dichlororhodamine dyes); US 4,318,846 (ether-substituted fluorescein dyes); US 5,800,996 (energy transfer dyes); US 5,066,580 (xanthine dyes); and US 5,688,648 (energy transfer dyes). Labelling can also be carried out with quantum dots, as described in US 6,322,901, US 6,576,291, US 6,423,551, US 6,251,303, US 6,319,426, US 6,426,513, US 6,444,143, US 5,990,479, US 6,207,392, US 2002/0045045 and US 2003/0017264. As used herein, the term "fluorescent label" comprises a signaling moiety that conveys information through the fluorescent absorption and/or emission properties of one or more molecules. Exemplary fluorescent properties comprise fluorescence intensity, fluorescence lifetime, emission spectrum characteristics and energy transfer.

Examples of commercially available fluorescent nucleotide analogues readily incorporated into nucleotide and/or polynucleotide sequences comprise, but are not limited to, Cy3-dCTP, Cy3-dUTP, Cy5-dCTP, Cy5-dUTP (Amersham Biosciences, Piscataway, N.J.), fluorescein- !2-dUTP, tetramethylrhodamine-6-dUTP, TEXAS RED^{™}-5-dUTP, CASCADE BLUE^{™}-7-dUTP, BODIPY TMFL-14-dUTP, BODIPY TMR-14-dUTP, BODIPY TMTR-14-dUTP, RHOD AMINE GREEN^{™}-5-dUTP, OREGON GREENR^{™} 488-5-dUTP, TEXAS RED^{™}-l2-dUTP, BODIPY^{™} 630/650-14-dUTP, BODIPY^{™} 650/665-14-dUTP, ALEXA FLUOR^{™} 488-5-dUTP, ALEXA FLUOR^{™} 532-5-dUTP, ALEXA FLUOR^{™} 568-5-dUTP, ALEXA FLUOR^{™} 594-5-dUTP, ALEXA FLUOR^{™} 546-14-dUTP, fluorescein- 12-UTP, tetramethylrhodamine-6-UTP, TEXAS RED^{™}-5- UTP, mCherry, CASCADE BLUE^{™}-7-UTP, BODIPY^{™} FL-14-UTP, BODIPY TMR- 14-UTP, BODIPY^{™} TR-14-UTP, RHOD AMINE GREEN^{™}-5-UTP, ALEXA FLUOR^{™} 488-5-UTP, and ALEXA FLUOR^{™} 546-14-UTP (Molecular Probes, Inc. Eugene, Oreg.). Methods for custom synthesis of nucleotides having other fluorophores include those described in Henegariu et al. (2000) Nature Biotechnol. 18:345.

Other fluorophores available for post-synthetic attachment comprise, but are not limited to, ALEXA FLUOR^{™} 350, ALEXA FLUOR^{™} 532, ALEXA FLUOR^{™} 546, ALEXA FLUOR^{™} 568, ALEXA FLUOR^{™} 594, ALEXA FLUOR^{™} 647, BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethyl rhodamine, Texas Red (available from Molecular Probes, Inc., Eugene, Oreg.), Cy2, Cy3.5, Cy5.5, and Cy7 (Amersham Biosciences, Piscataway, N.J.). FRET tandem fluorophores may also be used, comprising, but not limited to, PerCP-Cy5.5, PE-Cy5, PE-Cy5.5, PE-Cy7, PE-Texas Red, APC-Cy7, PE-Alexa dyes (610, 647, 680), and APC-Alexa dyes.

In some cases, metallic silver or gold particles may be used to enhance signal from fluorescently labeled nucleotide and/or polynucleotide sequences (Lakowicz et al. (2003) Bio Techniques 34:62).

Biotin, or a derivative thereof, may also be used as a label on a nucleotide and/or a polynucleotide sequence, and subsequently bound by a detectably labeled avidin/streptavidin derivative (e.g., phycoerythrin-conjugated streptavidin), or a detectably labeled anti-biotin antibody. Digoxigenin may be incorporated as a label and subsequently bound by a detectably labeled anti-digoxigenin antibody (e.g., fluoresceinated anti-digoxigenin). An aminoallyl-dUTP residue may be incorporated into a polynucleotide sequence and subsequently coupled to an N-hydroxy succinimide (NHS) derivatized fluorescent dye. In general, any member of a conjugate pair may be incorporated into a detection polynucleotide provided that a detectably labeled conjugate partner can be bound to permit detection. As used herein, the term antibody refers to an antibody molecule of any class, or any sub-fragment thereof, such as an Fab.

Other suitable labels for a polynucleotide sequence may comprise fluorescein (FAM), digoxigenin, dinitrophenol (DNP), dansyl, biotin, bromodeoxyuridine (BrdU), hexahistidine (6xHis), and phosphor-amino acids (e.g., P-tyr, P-ser, P-thr). In some embodiments the following hapten/antibody pairs are used for detection, in which each of the antibodies is derivatized with a detectable label: biotin/a-biotin, digoxigenin/a- digoxigenin, dinitrophenol (DNP)/a-DNP, 5-Carboxyfluorescein (FAM)/a-FAM.

In some embodiments, a nucleotide and/or a oligonucleotide sequence can be indirectly labeled, especially with a hapten that is then bound by a capture agent, e.g., as disclosed in US 5,344,757, US 5,702,888, US 5,354,657, US 5,198,537 and US 4,849,336, and PCT publication WO 91/17160. Many different hapten-capture agent pairs are available for use. Exemplary haptens comprise, but are not limited to, biotin, des-biotin and other derivatives, dinitrophenol, dansyl, fluorescein, Cy5, and digoxigenin. For biotin, a capture agent may be avidin, streptavidin, or antibodies. Antibodies may be used as capture agents for the other haptens (many dye-antibody pairs being commercially available, e.g., Molecular Probes, Eugene, Oreg.).

In some aspects, the detecting involves using detection methods such as flow cytometry; sequencing; probe binding and electrochemical detection; pH alteration; catalysis induced by enzymes bound to DNA tags; quantum entanglement; Raman spectroscopy; terahertz wave technology; and/or scanning electron microscopy. In some aspects, the flow cytometry is mass cytometry or fluorescence-activated flow cytometry. In some aspects, the detecting comprises performing microscopy, scanning mass spectrometry or other imaging techniques described herein. In such aspects, the detecting comprises determining a signal, e.g., a fluorescent signal.

In some aspects, the detection (comprising imaging) is carried out using any of a number of different types of microscopy, e.g., confocal microscopy, two-photon microscopy, light-field microscopy, intact tissue expansion microscopy, and/or CLARITY^{™}-optimized light sheet microscopy (COLM).

In some embodiments, fluorescence microscopy is used for detection and imaging of the detectable probe. In some aspects, a fluorescence microscope is an optical microscope that uses fluorescence and phosphorescence instead of, or in addition to, reflection and absorption to study properties of organic or inorganic substances. In fluorescence microscopy, a sample is illuminated with light of a wavelength which excites fluorescence in the sample. The fluoresced light, which is usually at a longer wavelength than the illumination, is then imaged through a microscope objective. Two filters may be used in this technique; an illumination (or excitation) filter which ensures the illumination is near monochromatic and at the correct wavelength, and a second emission (or barrier) filter which ensures none of the excitation light source reaches the detector. Alternatively, these functions may both be accomplished by a single dichroic filter. The "fluorescence microscope" comprises any microscope that uses fluorescence to generate an image, whether it is a more simple set up like an epifluorescence microscope, or a more complicated design such as a confocal microscope, which uses optical sectioning to get better resolution of the fluorescent image.

In some embodiments, confocal microscopy is used for detection and imaging of the detectable probe. Confocal microscopy uses point illumination and a pinhole in an optically conjugate plane in front of the detector to eliminate out-of-focus signal. As only light produced by fluorescence very close to the focal plane can be detected, the image's optical resolution, particularly in the sample depth direction, is much better than that of wide-field microscopes. However, as much of the light from sample fluorescence is blocked at the pinhole, this increased resolution is at the cost of decreased signal intensity - so long exposures are often required. As only one point in the sample is illuminated at a time, 2D or 3D imaging requires scanning over a regular raster (i.e., a rectangular pattern of parallel scanning lines) in the specimen. The achievable thickness of the focal plane is defined mostly by the wavelength of the used light divided by the numerical aperture of the objective lens, but also by the optical properties of the specimen. The thin optical sectioning possible makes these types of microscopes particularly good at 3D imaging and surface profiling of samples. CLARITY^{™}-optimized light sheet microscopy (COLM) provides an alternative microscopy for fast 3D imaging of large clarified samples. COLM interrogates large immunostained tissues, permits increased speed of acquisition and results in a higher quality of generated data.

Other types of microscopy that can be employed comprise bright field microscopy, oblique illumination microscopy, dark field microscopy, phase contrast, differential interference contrast (DIC) microscopy, interference reflection microscopy (also known as reflected interference contrast, or RIC), single plane illumination microscopy (SPIM), super-resolution microscopy, laser microscopy, electron microscopy (EM), Transmission electron microscopy (TEM), Scanning electron microscopy (SEM), reflection electron microscopy (REM), Scanning transmission electron microscopy (STEM) and low-voltage electron microscopy (LVEM), scanning probe microscopy (SPM), atomic force microscopy (ATM), ballistic electron emission microscopy (BEEM), chemical force microscopy (CFM), conductive atomic force microscopy (C- AFM), electrochemical scanning tunneling microscope (ECSTM), electrostatic force microscopy (EFM), fluidic force microscope (FluidFM), force modulation microscopy (FMM), feature-oriented scanning probe microscopy (FOSPM), kelvin probe force microscopy (KPFM), magnetic force microscopy (MFM), magnetic resonance force microscopy (MRFM), near-field scanning optical microscopy (NSOM) (or SNOM, scanning near-field optical microscopy, SNOM, Piezoresponse Force Microscopy (PFM), PSTM, photon scanning tunneling microscopy (PSTM), PTMS, photothermal microspectroscopy/ microscopy (PTMS), SCM, scanning capacitance microscopy (SCM), SECM, scanning electrochemical microscopy (SECM), SGM, scanning gate microscopy (SGM), SHPM, scanning Hall probe microscopy (SHPM), SICM, scanning ion-conductance microscopy (SICM), SPSM spin polarized scanning tunneling microscopy (SPSM), SSRM, scanning spreading resistance microscopy (SSRM), SThM, scanning thermal microscopy (SThM), STM, scanning tunneling microscopy (STM), STP, scanning tunneling potentiometry (STP), SVM, scanning voltage microscopy (SVM), and synchrotron x-ray scanning tunneling microscopy (SXSTM), and intact tissue expansion microscopy (exM).

In some embodiments, sequencing can be performed *in situ* and such sequencing methods typically involve incorporation of a labeled nucleotide (e.g., fluorescently labeled mononucleotides or dinucleotides) in a sequential, template-dependent manner or hybridization of a labeled primer (e.g., a labeled random hexamer) to a nucleic acid template such that the identities (i.e., nucleotide sequence) of the incorporated nucleotides or labeled primer extension products can be determined, and consequently, the nucleotide sequence of the corresponding template nucleic acid. Aspects of *in situ* sequencing are described, for example, in Mitra et al., (2003) Anal. Biochem. 320, 55-65, and Lee et al., (2014) Science, 343(6177), 1360-1363. In addition, examples of methods and systems for performing *in situ* sequencing are described in US 2016/0024555, US 2019/0194709, and in US 10,138,509, US 10,494,662 and US 10,179,932. Exemplary techniques for in situ sequencing comprise, but are not limited to, STARmap (described for example in Wang et al., (2018) Science, 361(6499) 5691), MERFISH (described for example in Moffitt, (2016) Methods in Enzymology, 572, 1-49), hybridization-based *in situ* sequencing (HybISS) (described for example in Gyllborg et al., Nucleic Acids Res (2020) 48(19):e112), and FISSEQ (described for example in US 2019/0032121).

In some embodiments, sequencing can be performed by sequencing-by-synthesis (SBS). In some embodiments, a sequencing primer is complementary to sequences at or near the one or more barcode(s). In such embodiments, sequencing-by-synthesis can comprise reverse transcription and/or amplification in order to generate a template sequence from which a primer sequence can bind. Exemplary SBS methods comprise those described for example, but not limited to, US 2007/0166705, US 2006/0188901, US 7,057,026, US 2006/0240439, US 2006/0281109, US 2011/0059865, US 2005/0100900, US 9,217,178, US 2009/0118128, US 2012/0270305, US 2013/0260372, and US 2013/0079232.

In some embodiments, sequencing can be performed by sequential fluorescence hybridization (e.g., sequencing by hybridization). Sequential fluorescence hybridization can involve sequential hybridization of detectable probes comprising an oligonucleotide and a detectable label.

In some embodiments, sequencing can be performed using single molecule sequencing by ligation. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. Aspects and features involved in sequencing by ligation are described, for example, in Shendure et al. Science (2005), 309: 1728-1732, and in US 5,599,675; US 5,750,341; US 6,969,488; US 6,172,218; US and 6,306,597.

In some embodiments, the barcodes of a probe or product thereof can be targeted by one or more other probes, such as unlabeled intermediate probes (which may be targeted by detectably labeled probes) or detectably labeled probes (e.g., fluorescently labeled oligonucleotides). In some embodiments, one or more decoding schemes are used to decode the signals, such as fluorescence, for sequence determination. In any of the embodiments herein, barcodes can be analyzed (e.g., detected or sequenced) using any suitable methods or techniques, comprising those described herein, such as RNA sequential probing of targets (RNA SPOTs), sequential fluorescent *in situ* hybridization (seqFISH), single-molecule fluorescent *in situ* hybridization (smFISH), multiplexed error-robust fluorescence *in situ* hybridization (MERFISH), hybridization-based *in situ* sequencing (HybISS), *in situ* sequencing, targeted *in situ* sequencing, fluorescent *in situ* sequencing (FISSEQ), or spatially-resolved transcript amplicon readout mapping (STARmap). In some embodiments, the methods provided herein comprise analyzing the barcodes by sequential hybridization and detection with a plurality of labelled probes (e.g., detection oligonucleotides). Exemplary decoding schemes are described in Eng et al., "Transcriptome-scale Super-Resolved Imaging in Tissues by RNA SeqFISH+," Nature 568(7751):235-239 (2019); Chen et al., "Spatially resolved, highly multiplexed RNA profiling in single cells," Science; 348(6233):aaa6090 (2015); Gyllborg et al., Nucleic Acids Res (2020) 48(19):e112; US 10,457,980 B2; US 2016/0369329 A1; WO 2018/026873 A1; and US 2017/0220733 A1, all of which are incorporated by reference in their entirety. In some embodiments, these assays enable signal amplification, combinatorial decoding, and error correction schemes at the same time.

In some embodiments, nucleic acid hybridization can be used for sequencing. These methods utilize labeled nucleic acid decoder probes that are complementary to at least a portion of a barcode sequence. Multiplex decoding can be performed with pools of many different probes with distinguishable labels. Non-limiting examples of nucleic acid hybridization sequencing are described for example in US 8,460,865, and in Gunderson et al., Genome Research 14:870-877 (2004).

In some embodiments, real-time monitoring of DNA polymerase activity can be used during sequencing. For example, nucleotide incorporations can be detected through fluorescence resonance energy transfer (FRET), as described for example in Levene et al., Science (2003), 299, 682-686, Lundquist et al., Opt. Lett. (2008), 33, 1026-1028, and Korlach et al., Proc. Natl. Acad. Sci. USA (2008), 105, 1176-1181.

In some aspects, the analysis and/or sequence determination can be carried out at room temperature for best preservation of tissue morphology with low background noise and error reduction. In some embodiments, the analysis and/or sequence determination comprises eliminating error accumulation as sequencing proceeds.

In some embodiments, the analysis and/or sequence determination involves washing to remove unbound polynucleotides, thereafter revealing a fluorescent product for imaging.

### VII. KITS

Also provided herein are kits for performing the methods of preparing three-dimensional polymerized matrices and/or for embedding a biological sample in a three-dimensional polymerized matrix. For example, in one aspect, provided herein is a kit for preparing a three-dimensional polymerized matrix, comprising a first planar substrate, a second planar substrate, one or more spacers (e.g., adhesive tape or rigid microparticles), one or more matrix-forming materials (e.g., hydrogel monomers), and instructions for performing the methods provided herein.

In still other embodiments, the kits further comprise one or more reagents for performing the analytical methods provided herein. In some embodiments, the kits further comprise one or more reagents required for one or more steps comprising hybridization, ligation, extension, detection, sequencing, and/or sample preparation as described herein. In some embodiments, the kit further comprises a target nucleic acid, e.g., any described in Section III. In some embodiments, any or all of the oligonucleotides are DNA molecules. In some embodiments, the target nucleic acid is a messenger RNA molecule. In some embodiments, the target nucleic acid is a probe (e.g., a padlock probe) or an amplification product thereof (e.g., a rolling circle amplification product, such as a nucleic acid concatemer). The various components of the kit may be present in separate containers or certain compatible components may be precombined into a single container. In some embodiments, the kits further contain instructions for using the components of the kit to practice the provided methods.

In some embodiments, the kits can contain reagents and/or consumables required for performing one or more steps of the provided methods. In some embodiments, the kits contain reagents for fixing, embedding, and/or permeabilizing the biological sample. In some embodiments, the kits contain reagents, such as enzymes and buffers for ligation and/or amplification, such as ligases and/or polymerases. In some aspects, the kit can also comprise any of the reagents described herein, e.g., wash buffer and ligation buffer. In some embodiments, the kits contain reagents for detection and/or sequencing, such as detectable probes or detectable labels. In some embodiments, the kits optionally contain other components, for example nucleic acid primers, enzymes and reagents, buffers, nucleotides, photoreactive nucleotides, and reagents for additional assays.

### VIII. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms "polynucleotide," "polynucleotide," and "nucleic acid molecule", used interchangeably herein, refer to polymeric forms of nucleotides of any length, either ribonucleotides or deoxyribonucleotides. Thus, this term comprises, but is not limited to, single-, double-, or multi- stranded DNA or RNA, genomic DNA, cDNA, DNA-RNA hybrids, or a polymer comprising purine and pyrimidine bases or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups.

"Hybridization" as used herein may refer to the process in which two single-stranded polynucleotides bind non-covalently to form a stable double-stranded polynucleotide. In one aspect, the resulting double-stranded polynucleotide can be a "hybrid" or "duplex." "Hybridization conditions" typically include salt concentrations of approximately less than 1 M, often less than about 500 mM and may be less than about 200 mM. A "hybridization buffer" includes a buffered salt solution such as 5% SSPE, or other such buffers. Hybridization temperatures can be as low as 5°C, but are typically greater than 22°C, and more typically greater than about 30°C, and typically in excess of 37°C. Hybridizations are often performed under stringent conditions, *i.e.,* conditions under which a sequence will hybridize to its target sequence but will not hybridize to other, non-complementary sequences. Stringent conditions are sequence-dependent and are different in different circumstances. For example, longer fragments may require higher hybridization temperatures for specific hybridization than short fragments. As other factors may affect the stringency of hybridization, including base composition and length of the complementary strands, presence of organic solvents, and the extent of base mismatching, the combination of parameters is more important than the absolute measure of any one parameter alone. Generally stringent conditions are selected to be about 5°C lower than the T*ₘ* for the specific sequence at a defined ionic strength and pH. The melting temperature T*ₘ* can be the temperature at which a population of double-stranded nucleic acid molecules becomes half dissociated into single strands. As indicated by standard references, a simple estimate of the T*ₘ* value may be calculated by the equation, T*ₘ* =81.5 + 0.41 (% G + C), when a nucleic acid is in aqueous solution at 1 M NaCl (see e.g., Anderson and Young, Quantitative Filter Hybridization, in Nucleic Acid Hybridization (1985)). Other references (*e.g.,* Allawi and SantaLucia, Jr., Biochemistry, 36:10581-94 (1997)) include alternative methods of computation which take structural and environmental, as well as sequence characteristics into account for the calculation of T*ₘ*.

In general, the stability of a hybrid is a function of the ion concentration and temperature. Typically, a hybridization reaction is performed under conditions of lower stringency, followed by washes of varying, but higher, stringency. Exemplary stringent conditions include a salt concentration of at least 0.01 M to no more than 1 M sodium ion concentration (or other salt) at a pH of about 7.0 to about 8.3 and a temperature of at least 25°C. For example, conditions of 5 × SSPE (750 mM NaCl, 50 mM sodium phosphate, 5 mM EDTA at pH 7.4) and a temperature of approximately 30°C are suitable for allele-specific hybridizations, though a suitable temperature depends on the length and/or GC content of the region hybridized. In one aspect, "stringency of hybridization" in determining percentage mismatch can be as follows: 1) high stringency: 0.1 × SSPE, 0.1% SDS, 65°C; 2) medium stringency: 0.2 × SSPE, 0.1% SDS, 50°C (also referred to as moderate stringency); and 3) low stringency: 1.0 × SSPE, 0.1% SDS, 50°C. It is understood that equivalent stringencies may be achieved using alternative buffers, salts and temperatures. For example, moderately stringent hybridization can refer to conditions that permit a nucleic acid molecule such as a probe to bind a complementary nucleic acid molecule. The hybridized nucleic acid molecules generally have at least 60% identity, including for example at least any of 70%, 75%, 80%, 85%, 90%, or 95% identity. Moderately stringent conditions can be conditions equivalent to hybridization in 50% formamide, 5 × Denhardt's solution, 5x SSPE, 0.2% SDS at 42°C, followed by washing in 0.2 × SSPE, 0.2% SDS, at 42°C. High stringency conditions can be provided, for example, by hybridization in 50% formamide, 5 × Denhardt's solution, 5 × SSPE, 0.2% SDS at 42°C, followed by washing in 0.1 × SSPE, and 0.1% SDS at 65°C. Low stringency hybridization can refer to conditions equivalent to hybridization in 10% formamide, 5 × Denhardt's solution, 6 × SSPE, 0.2% SDS at 22°C, followed by washing in 1x SSPE, 0.2% SDS, at 37°C. Denhardt's solution contains 1% Ficoll, 1% polyvinylpyrolidone, and 1% bovine serum albumin (BSA). 20 × SSPE (sodium chloride, sodium phosphate, ethylene diamide tetraacetic acid (EDTA)) contains 3M sodium chloride, 0.2M sodium phosphate, and 0.025 M EDTA. Other suitable moderate stringency and high stringency hybridization buffers and conditions are described, for example, in Sambrook et al., Molecular Cloning: A Laboratory Manual, 2nd ed., Cold Spring Harbor Press, Plainview, N.Y. (1989); and Ausubel et al., Short Protocols in Molecular Biology, 4th ed., John Wiley & Sons (1999).

Alternatively, substantial complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. *See* M. Kanehisa, Nucleic Acids Res. 12:203 (1984).

A "primer" used herein can be an oligonucleotide, either natural or synthetic, that is capable, upon forming a duplex with a polynucleotide template, of acting as a point of initiation of nucleic acid synthesis and being extended from its 3' end along the template so that an extended duplex is formed. The sequence of nucleotides added during the extension process is determined by the sequence of the template polynucleotide. Primers usually are extended by a DNA polymerase.

"Ligation" may refer to the formation of a covalent bond or linkage between the termini of two or more nucleic acids, e.g., oligonucleotides and/or polynucleotides, in a template-driven reaction. The nature of the bond or linkage may vary widely and the ligation may be carried out enzymatically or chemically. As used herein, ligations are usually carried out enzymatically to form a phosphodiester linkage between a 5' carbon terminal nucleotide of one oligonucleotide with a 3' carbon of another nucleotide.

"Sequencing," "sequence determination" and the like means determination of information relating to the nucleotide base sequence of a nucleic acid. Such information may include the identification or determination of partial as well as full sequence information of the nucleic acid. Sequence information may be determined with varying degrees of statistical reliability or confidence. In one aspect, the term includes the determination of the identity and ordering of a plurality of contiguous nucleotides in a nucleic acid. "High throughput digital sequencing" or "next generation sequencing" means sequence determination using methods that determine many (typically thousands to billions) of nucleic acid sequences in an intrinsically parallel manner, i.e. where DNA templates are prepared for sequencing not one at a time, but in a bulk process, and where many sequences are read out preferably in parallel, or alternatively using an ultra-high throughput serial process that itself may be parallelized. Such methods include but are not limited to pyrosequencing (for example, as commercialized by 454 Life Sciences, Inc., Branford, Conn.); sequencing by ligation (for example, as commercialized in the SOLiD^{™} technology, Life Technologies, Inc., Carlsbad, Calif.); sequencing by synthesis using modified nucleotides (such as commercialized in TruSeq^{™} and HiSeq^{™} technology by Illumina, Inc., San Diego, Calif.; HeliScope^{™} by Helicos Biosciences Corporation, Cambridge, Ma.; and PacBio RS by Pacific Biosciences of California, Inc., Menlo Park, Calif.), sequencing by ion detection technologies (such as Ion Torrent^{™} technology, Life Technologies, Carlsbad, Calif.); sequencing of DNA nanoballs (Complete Genomics, Inc., Mountain View, Calif.); nanopore-based sequencing technologies (for example, as developed by Oxford Nanopore Technologies, LTD, Oxford, UK), and like highly parallelized sequencing methods.

"Multiplexing" or "multiplex assay" herein may refer to an assay or other analytical method in which the presence and/or amount of multiple targets, e.g., multiple nucleic acid target sequences, can be assayed simultaneously by using more than one capture probe conjugate, each of which has at least one different detection characteristic, e.g., fluorescence characteristic (for example excitation wavelength, emission wavelength, emission intensity, FWHM (full width at half maximum peak height), or fluorescence lifetime) or a unique nucleic acid or protein sequence characteristic.

The term "about" as used herein refers to the usual error range for the respective value readily known to the skilled person in this technical field. Reference to "about" a value or parameter herein comprises (and describes) embodiments that are directed to that value or parameter per se.

As used herein, the singular forms "a," "an," and "the" comprise plural referents unless the context clearly dictates otherwise. For example, "a" or "an" means "at least one" or "one or more."

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be comprised in the smaller ranges, and are also encompassed within the claimed subject matter, subject to any specifically excluded limit in the stated range. Where the stated range comprises one or both of the limits, ranges excluding either or both of those comprised limits are also comprised in the claimed subject matter. This applies regardless of the breadth of the range.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b), etc., or i), ii), etc. does not by itself connote any priority, precedence, or order of steps in the claims. Similarly, the use of these terms in the specification does not by itself connote any required priority, precedence, or order.

### EXAMPLES

The presently disclosed subject matter will be better understood by reference to the following Examples, which are provided as exemplary of the disclosure, and not by way of limitation.

### Example 1: Preparation of Thin Hydrogel Matrix

The present example details exemplary protocols for preparing three-dimensional polymerized matrices (hydrogel matrices). Hydrogel matrices prepared using adhesive tape applied to a flat casting cap or silica monospheres applied to a glass slide to control the thickness were compared to hydrogel matrices prepared with a commercially available machined casting mold with a pedestaled cap or, alternatively, flat cap without any spacer. The hydrogel matrices prepared in this example were further subjected to fluorescence microscopy to determine the thicknesses achievable with the methods.

### Preparation of Hydrogel Matrix and Spacers

A master hydrogel mixture was prepared, using a 19:1:2 weight ratio of acrylamide (AM) to polydopamine (PDA) to N,N'-diallyltartardiamide (DATD). The master mixture was diluted in a buffer solution to provide a standard hydrogel mixture to be used for the comparison. TetraSpeck^{™} beads (0.2 µm) diameter were added to the standard gel mixture at a 1:100 weight ratio, to allow for later determination of hydrogel thickness.

### Preparation of Spacers and Glass Slides

Five clean slides coated with 3-(Trimethoxysilyl)propyl methacrylate (TMSPMA) for enhanced tissue adhesion were obtained. Plastic casting mold bases were attached to each of the clean slides, with each base leaving two exposed regions of the slide, where two complementary individual casting caps could be placed, as shown in FIG. 2A.

For the adhesive tape samples: either a pre-fabricated mask tape (thickness ~200 µm) as shown in FIG. 3A or individual squares of adhesive transfer tape (thickness ~10-20 µm) as shown in FIG. 3B were applied to the bottom of four flat casting caps (without pedestals), with two caps modified by mask tape and two caps modified by transfer tape. The adhesive tape-modified flat casting caps were placed in contact with the exposed regions of two slides, with the adhesive tape-modified end of each casting cap in contact with the slide. Standard gel mixture was injected into the region between the casting cap and the glass slide.

For the silica monospheres: standard gel mixture was applied to the exposed regions of two slides. After the standard gel mixture was applied, a suspension of silica monospheres (monodisperse, non-porous, 20 µm) in mineral oil was applied to the same surface around outer edges of the gel mixture within the exposed region. FIG. 4A provides an exemplary illustration of the application of silica monospheres around the gel mixture. After the silica monospheres had been applied, the two slides were set aside to allow for the mineral oil to evaporate. Then, after the mineral oil had completely evaporated, flat casting caps (without pedestals) were placed on top of the gel mixture.

For the control sample: flat casting caps (without pedestals) were placed on top of the exposed region of a single slide, and standard gel mixture was injected into the region between the casting cap and the glass slide.

The hydrogel mixtures for each sample were polymerized. After polymerization, the casting caps were removed to allow for thickness measurements of the hydrogels formed.

### Determination of Thickness of Three-Dimensional Polymerized Matrix

The hydrogel samples obtained above were each analyzed by fluorescence microscopy to determine the hydrogel thickness. For each sample, the positions of the top-most TetraSpeck^{™} bead as well as the bottom-most TetraSpeck^{™} bead were recorded in three x-y positions for each hydrogel sample. The thickness of the hydrogel samples were calculated as the difference between the position of the top-most bead and the position of the bottom-most bead in the gel. **Table 1 shows** the average measured thicknesses and standard deviation for each of the samples prepared using the various spacer materials. Sample #1 and sample #2 in **Table 1** refer to the two separate hydrogels prepared for a single slide, using the same spacer material.

**Table 1**

| **Spacer Type** | **Spacer Thickness (µm)** | **Gel thickness, sample #1 (µm)** | | **Gel thickness, sample #2 (µm)** | |
|---|---|---|---|---|---|
| | | **Average** | **Std. Dev.** | **Average** | **Std. Dev.** |
| Mask tape | ~100 | 93.0 | 7.6 | 87.1 | 6.3 |
| Transfer tape | 10-20 | 42.5 | 4.7 | 40.6 | 3.5 |
| Silica monosphere #1 | 20 | N/A | N/A | 21.2 | 11.7 |
| Silica monosphere #2 | 20 | 24.8 | 2.1 | 24.3 | 1.0 |
| No spacer | 0 | 3.3 | 1.8 | 8.5 | 3.4 |

As shown in **Table 1,** mask tape gave the thickest hydrogel matrices, with thicknesses below 100 µm. The use of transfer tape gave hydrogel matrices having half the gel thickness as the mask tape, and with only about 5 µm variation. The hydrogel matrices prepared using silica monospheres gave a further reduction in thickness by half, with thicknesses ~24 µm and variation of 1-2 µm. The control sample prepared using a flat casting cap without pedestals gave the thinnest hydrogel samples but showed high standard deviation relative to the thickness.

### Example 2: Preparation of Thin Hydrogel Matrix

The present example describes preparation of tissue samples embedded within an hydrogel matrices according the methods of the present disclosure, as compared to hydrogel matrices prepared with a casting cap with pedestals and a flat casting cap fitted with a round coverslip.

The methods compared in this example included the use of (i) a machined casting cap with feet, 200 µm; (ii) a flat casting cap with a round coverslip and adhesive tape (laser cut), 100 µm; (iii) a flat casting cap with a round coverslip and adhesive tape (scissor cut), 50 µm; (iv) a flat casting cap with round coverslip only, 50 µm; and (v) silica monospheres, 20 µm.

The methods employing the machined casting cap with pedestals (i) and the silica monospheres (v) were the same as described in Example 1 above. For the adhesive tape approaches (ii) and (iii), the method of applying the tape was as describe in Example 1 above but with the added insertion of a round coverslip between the flat casting cap and tape prior to application of the tape.

As an additional comparison showing hydrogels formed without spacers, a further method of using a round coverslip to provide surface tension with the hydrogel mixture and weight to extrude any excess hydrogel mixture, with the intent to reduce the hydrogel thickness, was employed. For the fourth method (iv), as shown in **FIG. 5A and 5B****,** a round, glass coverslip (type #2, thickness 0.17-0.25 mm; diameter 12 mm) was affixed to the surface of a flat, plastic casting cap. The casting cap with the round coverslip attached was placed on top of the hydrogel mixture with the coverslip in contact with the gel mixture.

The same standard gel mix and polymerization conditions as employed in Example 1 were used in the present example for all samples.

The materials and spacers used in the comparison are illustrated in the middle panel of **FIG. 6****.** The bottom panel of **FIG. 6** shows the resulting hydrogel matrices formed for each method and materials used.

### Example 3: Preparation of Tissue Sample Embedded in a Thin Hydrogel Matrix

The present example describes preparation of tissue samples embedded within an hydrogel matrices according the methods of the present disclosure, and measurement of tissue clearing times for the same.

Fixed, frozen mouse brain tissue samples were obtained for this example. The tissue samples were embedded in hydrogel matrices, prepared with (i) a standard casting mold with a pedestaled casting cap, 200 µm thickness; (ii) an adhesive tape mask tape applied to a flat casting cap, 70-100 µm thickness; (iii) round coverslip applied to a flat casting cap, 14-50 µm thickness; (iv) silica monospheres (monodisperse, non-porous), 20 µm diameter. A tissue sample fixed and permeabilized to a sample slide but not embedded in a hydrogel matrix was used as a control (v).

For the embedded tissue sample prepared with a standard casting mold with a pedestaled casting cap (~200 µm pedestals): the pre-machined casting cap with feet was placed in contact with the same surface of the glass slide to which the tissue sample was fixed, standard gel mixture was injected into the region between the casting cap and the glass slide, and the hydrogel mixture polymerized to form the hydrogel matrix around the fixed tissue sample.

The embedding protocol for the tissue samples using the mask tape and monospheres were prepared using the same protocol as described in Example 1 above, with the hydrogel mixture delivered to the tissue sample fixed to the surface of the glass slide (the region between the casting cap and the glass slide).

For the embedded tissue sample prepared with round coverslip, 14-50 µm thickness: the embedding protocol for the tissue samples was the same protocol as described in Example 2 above.

The same standard gel mix and polymerization conditions as employed in Example 1 were used in the present example for all samples.

After the tissue samples were embedded in their hydrogel matrices, each tissue sample was treated with clearing reagents (proteinase K and SDS) and washed to remove any tissue matrix (protein and lipids). Fluorescent images of the embedded samples were taken (recorded at wavelength 488 nm) before the samples were treated with the clearing reagents, as well as at 30 minutes (0.5 h), an hour (1 h), and two hours (2 h) after being treated with the clearing reagents. **FIG. 7A** shows the images taken at each time point after clearing. **FIG. 7B** depicts a plot of the residual tissue at each time point after clearing, in which the percentage of remaining fluorescence at each time point relative to the initial fluorescence was taken to represent the percentage of residual tissue for the given time point.

As shown in **FIG. 7A** and **FIG. 7B****,** the residual tissue left at various points after clearing depended upon the thickness of the hydrogel matrix in which the tissue sample was initially embedded. The tissue sample prepared using the pedestaled casting cap (~200 µm) showed the most residual tissue at each time point (i). The tissue samples embedded in hydrogel matrices prepared with mask tape (ii), the cover slip (iii) or silica monospheres (iv) all showed similar clearing rates and residual tissue at the same time points. The tissue sample that was not embedded in a hydrogel matrix (v) was cleared in less than 30 minutes and showed negligible change in residual tissue at later time points. In summary, the tissue samples prepared with the methods of the present disclosure have a thinner hydrogel matrix, which in turn allows for faster diffusion through the hydrogel material to the embedded tissue sample, which further results in shorter clearing times.

### Example 4: Analyte Detection in Thin Hydrogel Embedded Tissue Samples

Mouse brain tissue sample embedded in hydrogel matrices prepared with silica monospheres (monodisperse, non-porous), 20 µm diameter. Specifically, silica beads were suspend in ethanol (instead of mineral oil used in Example 1), and the beads were pipetted on corners of each well having a tissue sample. Using ethanol (miscible with hydrogel mixtures) avoided mineral oil residual on the samples. After drying of ethanol, a hydrogel mix was applied in the middle of the well to cover the tissue sample. Flat casting caps (without pedestals) were placed on top of a coverslip above the hydrogel mix, and polymerization of the hydrogel mix between the sample substrate and the coverslip generated a thin hydrogel embedding the tissue sample. The control tissue sample was not embedded in a hydrogel matrix. The samples were not cleared prior to probe hybridization.

Circularizable probes (e.g., padlock probes) targeting nucleic acid analytes of interest were added in hybridization buffers and incubated with the samples to allow hybridization of the circularizable probes to their target nucleic acids. Then, the probe hybridization mixture was removed and the samples were washed. For ligation of the circularizable probes hybridized to their target nucleic acids, a ligation reaction mix (e.g., containing a ligase and a ligase buffer) and a rolling circle amplification (RCA) primer were added to the samples and incubated for probe circularization and RCA primer hybridization to the probes. The samples were washed and an RCA reaction mixture (containing Phi29 reaction buffer, dNTPs, Phi29 polymerase) was added and incubated for RCA of the circularized probes. The RCPs in the thin hydrogel embedded sample were tethered to the hydrogel matrix.

The samples were washed and detectable probes in a hybridization buffer were hybridized to RCA products (RCPs) in the sample. The detectable probes included probes that hybridize to sequences in the RCPs and comprise overhangs for hybridization of fluorescently labelled detection oligonucleotides. The samples were imaged using a fluorescent microscope to detect signals associated with the RCPs.

As shown in **FIG. 8****,** both the thin hydrogel embedded sample and the control showed good RCP signals, with the hydrogel embedded sample showing more RCP signals than the control. These data demonstrate that thin hydrogel embedding and RCP tethering to the hydrogel matrix were compatible with *in situ* detection of signals associated with analytes in the embedded samples.

The present disclosure is not intended to be limited in scope to the particular disclosed embodiments, which are provided, for example, to illustrate various aspects of the disclosure. Various modifications to the compositions and methods described will become apparent from the description and teachings herein. Such variations may be practiced without departing from the true scope and spirit of the disclosure and are intended to fall within the scope of the present disclosure.

### NUMBERED EMBODIMENTS

1. A method for preparing a three-dimensional polymerized matrix, comprising:
   a) delivering a matrix-forming material in a space between a surface of a first planar substrate and a surface of a second planar substrate, wherein:
      the space is at least partially enclosed by a spacer between the first and second planar substrates, and
      the spacer is non-integral to the first or second planar substrate; and
   b) forming a three-dimensional polymerized matrix from the matrix-forming material in the space, wherein the three-dimensional polymerized matrix has an average thickness between about 5 µm and about 200 µm.
2. The method of embodiment 1, further comprising c) removing the spacer from the first and/or the second planar substrates.
3. The method of embodiment 1 or embodiment 2, wherein the spacer is an adhesive tape, optionally wherein the adhesive tape has a thickness of about 100 µm or between about 10 µm and 20 µm.
4. The method of embodiment 3, wherein the distance between the surfaces of the first and second planar substrates is substantially equal to the thickness of the adhesive tape.
5. The method of embodiment 1 or embodiment 2, wherein the spacer comprises a plurality of microparticles, optionally wherein the average diameter of the rigid microparticles is between about 10 µm and 50 µm.
6. The method of embodiment 5, wherein the distance between the surfaces of the first and second planar substrates is substantially equal to the average diameter of the rigid microparticles.
7. The method of embodiment 5 or embodiment 6, wherein the plurality of microparticles is monodisperse.
8. The method of any one of embodiments 5 to 7, wherein the plurality of microparticles comprises a plurality of silica monospheres.
9. The method of any one of embodiments 1 to 8, further comprising:
   applying the spacer to the surface of the first planar substrate and/or the surface of the second planar substrate, and
   bringing the first planar substrate and second planar substrate together such that the spacer separates the first and second planar substrates to provide the space.
10. The method of embodiment 9, wherein the spacer comprises a plurality of microparticles, and the applying step comprises adding a suspension to the surface of either the first planar substrate or second planar substrate, wherein the suspension comprises the plurality of microparticles and a carrier.
11. The method of embodiment 10, wherein the carrier comprises mineral oil or an organic solvent, optionally wherein the organic solvent is ethanol.
12. The method of embodiment 10 or embodiment 11, wherein the plurality of microparticles are not mixed with the matrix-forming material and do not contact the three-dimensional polymerized matrix.
13. The method of any one of embodiments 1 to 12, wherein the three-dimensional polymerized matrix comprises a biological sample embedded therein.
14. The method of embodiment 13, wherein the biological sample is immobilized on the surface of the first planar substrate.
15. The method of embodiment 14, further comprising fixing the biological sample prior to the delivering step.
16. The method of any one of embodiments 13 to 15, wherein the first planar substrate comprises a coating with or is functionalized with one or more substances to facilitate attachment of the biological sample.
17. The method of embodiment 16, wherein the one or more substances comprise lectins, poly-lysine, antibodies, polysaccharides, or covalently binding moieties, optionally wherein the one or more substances comprises acryloyls.
18. The method of embodiment any one of embodiments 15 to 17, further comprising permeabilizing the biological sample after the fixing step and prior to the delivering step.
19. The method of any one of embodiments 13 to 18, further comprising cross-linking the biological sample embedded within the three-dimensional polymerized matrix.
20. The method of any one of embodiments 13 to 19, further comprising clearing the biological sample embedded within the three-dimensional polymerized matrix.
21. The method of any one of embodiments 13 to 20, wherein the biological sample is a tissue slice between about 1 µm and about 50 µm in thickness, optionally wherein the tissue slice is between about 5 µm and about 35 µm in thickness.
22. The method of any one of embodiments 1 to 21, wherein the matrix-forming material comprises a plurality of fluorescent beads, and wherein the thickness of the three-dimensional polymerized matrix is measured by imaging the fluorescent beads, optionally wherein the fluorescent beads have an average diameter of about 0.2 µm.
23. The method of any one of embodiments 1 to 22, further comprising applying a force to the first and/or second planar substrates prior to forming the three-dimensional polymerized matrix.
24. The method of any one of embodiments 1 to 23, wherein the three-dimensional polymerized matrix is formed by subjecting the matrix-forming material to polymerization.
25. The method of embodiment 24, wherein the polymerization is initiated by adding a polymerization-inducing catalyst, UV light or functional cross-linkers.
26. The method of any one of embodiments 1 to 25, wherein the matrix-forming material comprises polyacrylamide, cellulose, alginate, polyamide, cross-linked agarose, cross-linked dextran or cross-linked polyethylene glycol.
27. The method of any one of embodiments 1 to 26, wherein the surface of the first or second planar substrate has a recessed cavity.
28. The method of any one of embodiments 1 to 27, wherein the surface of the first or second planar substrate comprises one or more positional markers and/or fiducial markers.
29. A method for embedding a biological sample in a three-dimensional polymerized matrix, comprising:
   a) applying a spacer to a surface of a first planar substrate or a surface of a second planar substrate, wherein the spacer is non-integral to the first or second planar substrate, and wherein a biological sample is immobilized on the surface of a first planar substrate;
   b) bringing the first and second planar substrates together to form a space between the first and second substrates such that the biological sample is in the space at least partially enclosed by the spacer;
   c) delivering a matrix-forming material in the space; and
   d) forming a three-dimensional polymerized matrix from the matrix-forming material in the space, wherein the three-dimensional polymerized matrix has an average thickness between about10 µm and about 100 µm,
   thereby embedding the biological sample in the three-dimensional polymerized matrix.
30. The method of embodiment 29, wherein the spacer is applied to the second planar substrate which is downward facing, and the method further comprises e) removing the second planar substrate from the first planar substrate, thereby removing the spacer at least partially enclosing the biological sample.
31. The method of embodiment 29, wherein the spacer is applied to the first planar substrate which is upward facing, and the method further comprises e) removing the second planar substrate from the first planar substrate, and optionally further removing the spacer from the first planar substrate.
32. The method of any of embodiments 29-31, further comprising crosslinking the biological sample embedded in the three-dimensional polymerized matrix.
33. The method of any of embodiments 29-32, further comprising clearing the biological sample embedded within the three-dimensional polymerized matrix.
34. The method of embodiment 33, wherein the clearing is performed for less than about 60 minutes.
35. The method of embodiment 33, wherein the clearing is performed for less than about 30 minutes.

## Claims

1. A composition comprising a biological sample embedded in a three-dimensional polymerized matrix, wherein the three-dimensional polymerized matrix has an average thickness between about 5 µm and about 100 µm, and wherein the polymerized matrix is a hydrogel.

2. The composition of claim 1, wherein the three-dimensional polymerized matrix has an average thickness between about 10 µm and about 100 µm, between about 10 µm and about 20 µm, or between about 5 µm and about 20 µm.

3. The composition of claim 1 or claim 2, wherein the composition is obtainable by:
a) applying a spacer to a surface of a first planar substrate or a surface of a second planar substrate, wherein the spacer is non-integral to the first planar substrate or the second planar substrate, and wherein a biological sample is immobilized on the surface of the first planar substrate;
b) bringing the first planar substrate and the second planar substrate together to form a space between the first planar substrate and the second planar substrate that comprises the biological sample, wherein the space is at least partially enclosed by the spacer;
c) delivering a matrix-forming material to the space; and
d) forming a three-dimensional polymerized matrix from the matrix-forming material in the space, wherein the three-dimensional polymerized matrix has an average thickness between about 10 µm and about 100 µm,
thereby embedding the biological sample in the three-dimensional polymerized matrix.

4. The composition of any one of claims 1-3, wherein the biological sample is a tissue sample, such as a tissue section, or cells.

5. The composition of any one of claims 1-4, wherein the biological sample is a fixed biological sample.

6. The composition of any one of claims 1-5, wherein the composition further comprises an analyte labelling agent, optionally wherein the labelling agent comprises an analyte binding moiety and a reporter oligonucleotide.

7. The composition of any one of claims 1-6, wherein the composition further comprises a nucleic acid probe, optionally wherein the nucleic acid probe is a circular probe or circularizable probe, such as a padlock probe.

8. The composition of any one of claims 1-8, wherein the composition further comprises one or more clearing reagents.

9. The composition of claim 8, wherein the one or more clearing reagents comprises proteinase K and SDS.

10. A method of *in situ* detection, wherein the method comprises providing a composition of any one of claims 1-9, and detecting or determining a presence of one or more analytes in the biological sample embedded in the three-dimensional polymerized matrix.

11. The method of claim 10, wherein the method of *in situ* detection comprises *in situ* hybridization or *in situ* sequencing.

12. The method of claim 10 or claim 11, wherein the one or more analytes comprises a nucleic acid.

13. The method of any one of claims 10-12, wherein the method comprises detection of nucleic acid sequences *in situ* using hybridization of probes to the nucleic acid sequences and generation of amplified signals associated with the probes.

14. The method of any one of claims 10-13, wherein the method further comprises clearing the biological sample embedded in the three-dimensional polymerized matrix.

15. The method of claim 14, wherein the method comprises performing the clearing for less than about 30 minutes.
